# FASCICULE DE BREVET EUROPEEN

(11) **EP 0 513 316 B1**
(45) Date de publication et mention de la délivrance du brevet: **23.04.1997**
(21) Numéro de dépôt: 92901155.9
(22) Date de dépôt: 21.11.1991
(51) Int. Cl.: C12Q 1/68, C12N 9/64, C12N 15/57, A61K 45/05

(54) **MARQUEURS ANALYTIQUES POUR LE CANCER, NOTAMMENT POUR LE CANCER MALIN DU SEIN**
ANALYTISCHER MARKER FÜR KREBS, BESONDERS FÜR BÖSARTIGEN BRUSTKREBS
ANALYTICAL TRACERS FOR CANCER, PARTICULARLY MALIGNANT BREAST CANCER

(30) Priorité: 21.11.1990 GB 9025326
(43) Date de publication de la demande: 19.11.1992
(73) Titulaire: INSTITUT NATIONAL DE LA SANTE ET DE LA RECHERCHE MEDICALE (INSERM), 75654 Paris Cédex 13 (FR)
(72) Inventeur: BASSET, Paul, F-67000 Strasbourg (FR); BELLOCQ, Jean-Pierre, F-67200 Strasbourg (FR); CHAMBON, Pierre, F-67113 Blaesheim (FR)
(74) Mandataire: Sheard, Andrew Gregory
(86) Numéro de dépôt international: FR9100924
(87) Numéro de publication internationale: WO9209701

(56) Documents cités:
- Trends in Genetics, vol., 6, no. 4 April 1990, Amsterdam, NL; L.M. Matrisian: "Metalloproteinases and their inhibitors in matrix remodling", pages 121-,125, voir l article en entier (cité dans la demande).
- The American Journal of Pathology, vol., 136, no. 3, Mars 1990, C. Monteagudo et al.: "Immunohistochemical distribution of type IV collagenase in normal, benign and malignant breast tissue", pages 585-592, voir abrégé; page 591, lignes 5-25.
- Nature, vol., 348, 20 décembre 1990, Londres, GB; P. Basset et al.: "A novel metalloproteinase gene specifically expressed in stromal cells of breast carcinomas", pages 699-704, voir l'article en entier.
- Biochem. J., vol. 240, pages 913-916 (1986)
- J. Biol. Chem., vol 261, No. 14, page 6600-6605 (1986)

## Description

La présente invention porte sur des marqueurs enzymatiques associés à une tumeur.

En utilisant des séquences d'ADN codant pour la stromélysine-3, et des anticorps capables de se lier à la stromélysine-3, la présente invention propose des procédés de diagnostic du cancer, et plus spécifiquement, du cancer malin du sein.

Le nombre de décès de par le monde provoqués par le cancer chaque année continue à être un souci majeur, seuls quelques traitements étant disponibles pour des types spécifiques de cancer, et ceux-ci ne donnant pas de garantie absolue de succès. La plupart des traitements reposent sur une approche générale de type "à portée d'action large", détruisant les cellules à croissance rapide dans l'espoir que les cellules cancéreuses à croissance rapide ou bien succomberont au traitement, ou bien seront au moins réduites en nombre pour permettre au système du corps d'éliminer le reste.

La recherche de remède a été entravée par la découverte que différentes formes de cancer nécessitent des traitements différents. Etant donné que pratiquement toute partie du corps peut être affectée par un cancer, la tâche devient énorme.

Néanmoins, malgré leurs différences, les cancers présentent également un certain nombre d'analogies. La première de celles-ci est la croissance de tissus indifférenciés. Cependant, même cela n'est pas exact à 100%, en ce sens que certaines cellules cancéreuses manifestent effectivement un degré de différenciation, ce qui est le cas dans les cancers du système sexuel, tels que ceux du sein et du testicule, où les tumeurs peuvent être positives ou négatives aux récepteurs hormonaux. Le traitement de ces tumeurs dépend de l'état hormonal, et peut être aussi simple qu'une administration de l'antagoniste hormonal approprié, tel que le Tamoxifène (marque de fabrique).

Un autre facteur que la plupart des cancers ont en commun est que, pour qu'ils soient mortels, ils doivent donner des métastases. Jusqu'au moment où se produit une métastase, une tumeur, bien qu'elle puisse être maligne, est confinée dans une zone du corps. Ceci peut provoquer un malaise et/ou une douleur, ou même conduire à des symptômes plus graves, mais si elle peut être localisée, elle peut être retirée par voie chirurgicale et, si cela est fait avec un soin approprié, ne pas provoquer de problèmes ultérieurs.

Cependant, une fois qu'une métastase est installée, une résection chirurgicale peut retirer la tumeur parentale, mais les cellules cancéreuses ont envahi le corps, et seule une chimiothérapie, ou certaines formes particulières de thérapie de cible, ont une certaine chance de succès.

Ainsi, l'aptitude à envahir localement et à donner des métastases dans des organes distants de la tumeur primaire (progression de la tumeur) est l'évènement mortel au cours de la plupart des cancers. Une modification/ dégradation de la matrice extracellulaire (MEC) entourant la tumeur primaire, et des modifications des propriétés d'adhérence des cellules tumorales, sont connues pour être décisives pour la dissociation des cellules métastatiques à partir des cellules tumorales primaires (Liotta, Cancer Res. 46:1-7 (1986) ; Hart et al., Biochim. Biophys. Acta 989:65-84 (1989)).

L'angiogenèse de la tumeur est essentielle à la fois pour l'expansion de la tumeur primaire et l'étalement de la tumeur métastatique, et l'angiogenèse elle-même nécessite une dégradation de la MEC (Blood et al., Biochim. Biophys. Acta 1032:89-118 (1990)). De ce fait, la malignité est une maladie systémique dans laquelle des interactions entre les cellules néoplasiques et leur environnement jouent un rôle décisif pendant l'évolution du processus pathologique (Fidler, I.J., Cancer Metastasis Rev. 5:29-49 (1986)).

L'identification des modifications dans l'expression des gènes qui sont associées aux tumeurs malignes, incluant celles mises en jeu dans la progression de la tumeur, est clairement une condition préalable non seulement pour une compréhension totale du cancer, mais également pour développer de nouvelles thérapies rationnelles de lutte contre le cancer. Des mutations et/ou un contrôle anormal de l'expression de deux groupes de gènes cellulaires (les protooncogènes et les gènes suppresseurs de tumeurs) se son avérés conduire, dans un procédé à plusieurs étapes, à la perte du contrôle de la croissance normale et à l'acquisition du phénotype de la cellule transformée (Weinberg, R.A. Cancer Res. 49:3713-3721 (1989)). Cependant, les mécanismes moléculaires qui conduisent à une progression de la tumeur sont bien moins clairs (Nowell, P.C. Cancer Res. 46:2203-2207 (1986) ; Fidler, I.J. Cytometry 10:673-680 (1989)).

Ainsi, il se pose un problème supplémentaire dans le fait que les gènes caractéristiques des cellules cancéreuses sont très souvent des gènes hôtes qui sont exprimés de façon anormale. C'est très souvent le cas qu'un marqueur protéinique particulier pour un cancer donné soit surexprimé dans ce cancer, mais soit également exprimé partout ailleurs à travers le corps, bien qu'à des niveaux réduits.

Certaines des protéines associées aux cancers sont des enzymes qui rompent la matrice extracellulaire, qui est importante pour maintenir les cellules dans leur relation appropriée les unes par rapport aux autres. L'une de ces classes est constituée par les métalloprotéinases (MMPs) (Matrisian, L.M., Trends Genet. 6:121-125 (1990)), ainsi appelées parce qu'elles se lient avec le zinc. Cependant, aucune n'a été découverte comme permettant de diagnostiquer le cancer, ou toute tumeur particulière, bien que la présence de certaines puisse être indicatrice.

Les MMPs sont mises en jeu dans un certain nombre de processus physiologiques et pathologiques dans lesquels le remodelage de la MEC et la migration cellulaire sont impliqués, par exemple la morphogenèse et le développement embryonnaire, l'arthrite rhumatoïde, et l'invasion de la tumeur et les métastases. Des inhibiteurs de MMP sont connus comme étant capables de bloquer l'invasion de la tumeur et l'angiogenèse, qui sont décisives pour la progression de la tumeur, dans les modèles expérimentaux.

Tous les membres de la famille des métalloprotéinases de matrice sont des protéinases qui dégradent au moins un composant de la MEC, qui sont sécrétées dans une forme latente et nécessitent une activation, telle qu'une protéolyse (par exemple par la plasmine) pour devenir actives. Des collagénases interstitielles attaquent de façon spécifique les collagènes du tissu conjonctif (I à III), alors que les collagénases du type IV (72 kD et 92 kD) dégradent les collagènes présents dans la membrane basale et la fibronectine. Les stromélysines (transines) -1 et -2, et également pump-1, ont une spécificité de substrat bien plus large, dégradant les protéoglycanes, la laminine, la fibronectine et les collagènes (III à V).

Chez l'homme, la plupart des tumeurs malignes sont des carcinomes, et parmi les non-fumeuses, le cancer du sein est la cause principale de la mortalité par le cancer chez la femme (Willett, W., Nature 338:389-394 (1989)). L'expression de plusieurs oncogènes a été rapportée comme étant modifiée dans les cellules et tumeurs malignes du sein, mais aucun modèle particulier d'expression d'oncogène/gène suppresseur ne peut être associé logiquement au cancer du sein (Gullick, W.J., Prog. Growth Factor Res. 2:1-13 (1990)).

Cependant, les cellules néoplasiques des tumeurs du sein sont souvent noyées dans un stroma adipeux et mésenchymateux, ce qui peut également être important dans le contrôle de leur prolifération et dans leur aptitude à donner des métastases. Naturellement, il est connu que les cellules du stroma peuvent moduler, à la fois positivement et négativement, la croissance de l'épithélium mammaire normal (Salomon et al., dans Breast Cancer : Cellular and Molecular Biology (éds., Lippman, M.E. et Dickson, R.B.), pages 363-389 (Kluwer, Boston, (1988)), et que des interactions entre les composants de l'épithélium et du stroma peuvent influencer la carcinogenèse épithéliale de la glande mammaire (DeOme et al., Cancer Res. 38:2103-2111 (1978)).

L'existence de fibroblastes "activés" (Tremblay, G. Exp. Mol. Pathol. 31:248-260 (1979)) et/ou anormaux (Grey et al., Proc. Nat. Acad. Sci. USA 86:2438-2442 (1989)) dans les tumeurs malignes du sein a été émise comme hypothèse, et il a été proposé que le cancer du sein pourrait représenter un échappement partiel de la dépendance d'une exigence du stroma ou d'une réponse anormalement forte à un composant du stroma.

En raison de la nature du tissu cancéreux, il est habituellement relativement aisé d'établir une culture continue, ou lignée cellulaire, d'un cancer donné, un procédé qui rend facile l'étude des effets d'un régime de traitement donné. Un inconvénient significatif de tels systèmes réside dans leur nature-même - alors que le traitement d'essai établira s'il peut agir directement contre les cellules, il n'est en aucune manière certain de savoir quel effet le traitement aura in vivo, et l'analyse biochimique de telles lignées se fait inévitablement en l'absence du tissu entourant normalement la tumeur in vivo.

C'est un objectif de l'invention que d'identifier des gènes dont l'expression est accrue dans les carcinomes du sein, les carcinomes du sein étant considérés comme des cellules épithéliales malignes interagissant avec leur stroma environnant.

Les présents inventeurs ont maintenant découvert qu'une protéine non caractérisée jusqu'à présent peut permettre le diagnostic de certains cancers invasifs, en particulier les carcinomes du sein, les carcinomes des cellules squameuses de la tête et du cou et les carcinomes de la peau (du type cellules squameuses et du type cellules basales). La protéine appartient apparemment au groupe des métalloprotéinases, et est désignée ici comme étant la stromélysine-3.

### DESCRIPTION DES FIGURES

- Figure 1: Analyse Northern blot de l'ARN total provenant du carcinome du sein C1 et du fibroadénome F1
L'ARN total provenant des cellules du carcinome du sein C1 et du fibroadénome F1 a été sondé à l'aide de quatre sondes d'ADNc isolés de façon indépendante comme décrit à l'Exemple 1.
- Figure 2: Séquence nucléotidique de l'ADNc de la stromélysine-3
La séquence nucléotidique de l'ADNc et la séquence d'acides aminés déduite de la stromélysine-3 est présentée. En partant de l'extrémité 5', les séquences nucléotidiques soulignées correspondent à : le peptide signal putatif ; la séquence PRCGVPD caractéristique de la prométalloprotéinase, les résidus d'histidine conservés du domaine de liaison au zinc et la séquence signal poly(A⁺).

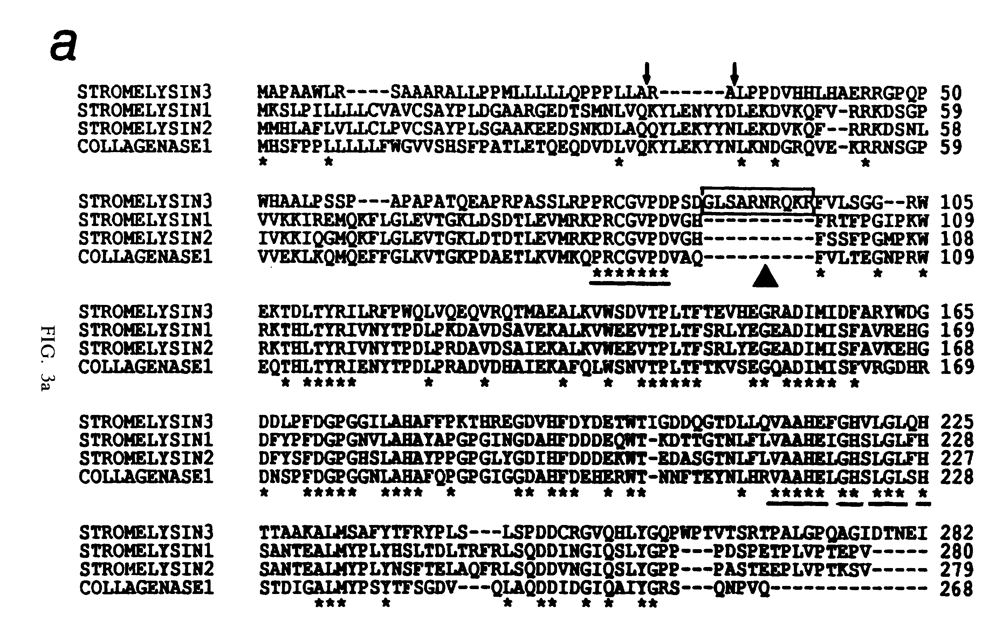

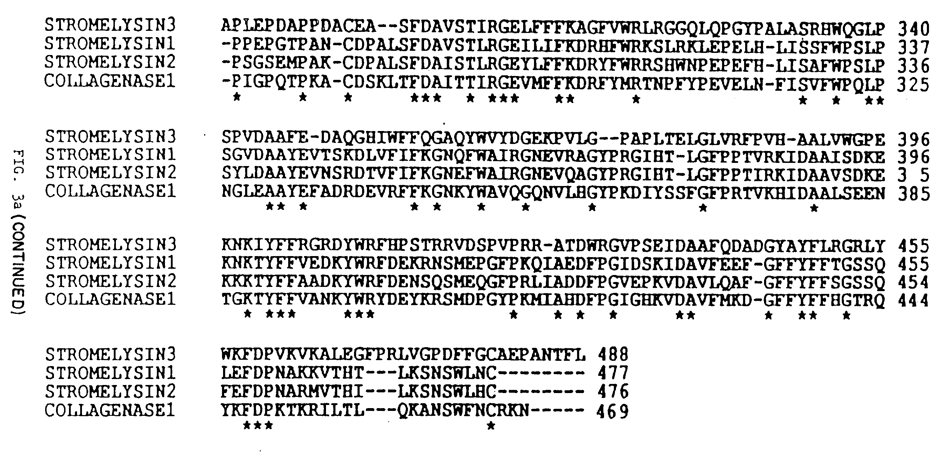

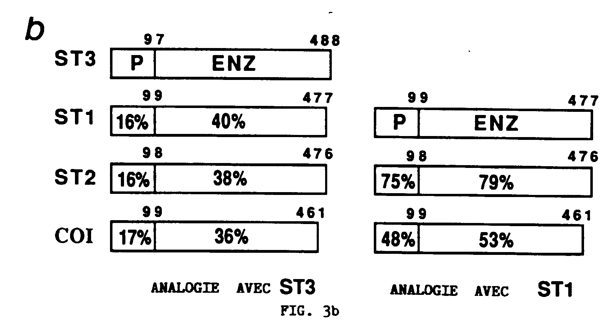

- Figure 3: Comparaison des séquences de métalloprotéinases
Les séquences d'acides aminés sont alignées et comparées pour la stromélysine-3, la stromélysine-2, la stromélysine-1 et la collagénase-1, toutes étant des métalloprotéinases supposées, comme décrit à l'Exemple 3.

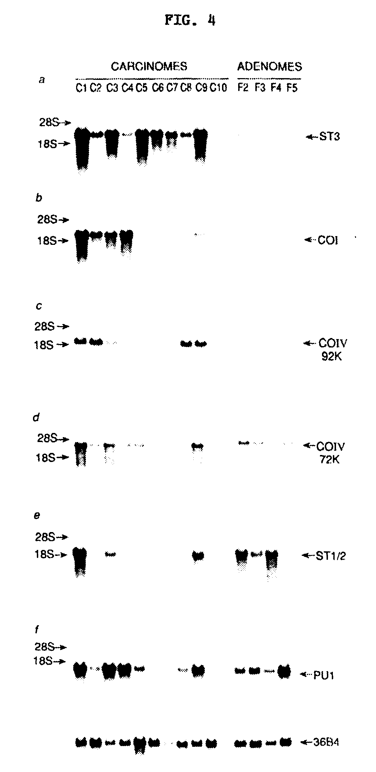

- Figure 4: Analyse Northern blot de la métalloprotéinase humaine
De l'ARN total a été préparé à partir de quatre carcinomes du sein négatifs aux récepteurs d'oestrogènes (C1, grade II ; C2, C3 et C4, grade III), six carcinomes du sein positifs aux récepteurs d'oestrogènes (C5, C8 et C9, grade II ; C6 et C7, grade III ; C10, grade I) et quatre fibroadénomes du sein (F2-F5).
L'ARN a été sondé avec (a) l'ARN de stromélysine-3, (b) l'ARN de collagénase type I (COI), (c) l'ARN de collagénase type IV de 92 kd (COIV 92 k), (d) l'ARN de collagénase type IV de 72 kd (COIV 72k), (e) l'ARN de stromélysine-1 et -2 (ST1/2) et l'ARN de pump-1 (PUI), comme décrit à l'Exemple 4.

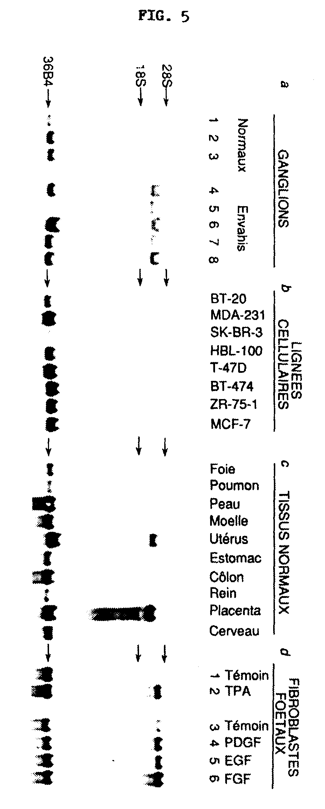

- Figure 5: Analyse par Northern blot de l'ARN de stromélysine-3 provenant de diverses lignées cellulaires et tissus
(a) Trois noeuds lymphatiques auxiliaires normaux et cinq métastatiques provenant de patientes atteintes d'un cancer du sein ; (b) quatre lignées de carcinome du sein négatives aux récepteurs d'oestrogènes (BT-20, MDA-231, SK-BR-3, HBL-100) et quatre positives aux récepteurs d'oestrogènes (T-47D, BT-474, ZR-75-1, MCF-7) ; (c) dix tissus, humains normaux ; et (d) des fibroblastes dérivés de foetus humain HFL-1 (ATCC CCL 153) cultivés dans du milieu exempt de sérum (1 et 2), en l'absence (1) ou en présence (2) de TPA, cultivés dans des milieux exempts de sérum supplémentés par 20 mg/ml d'insuline (3-6), en l'absence (3) ou en présence (4) de PDGF, (5) d'EGF, ou (6) de bFGF, ont été sondés avec des séquences de stromélysine-3, comme décrit à l'Exemple 5.

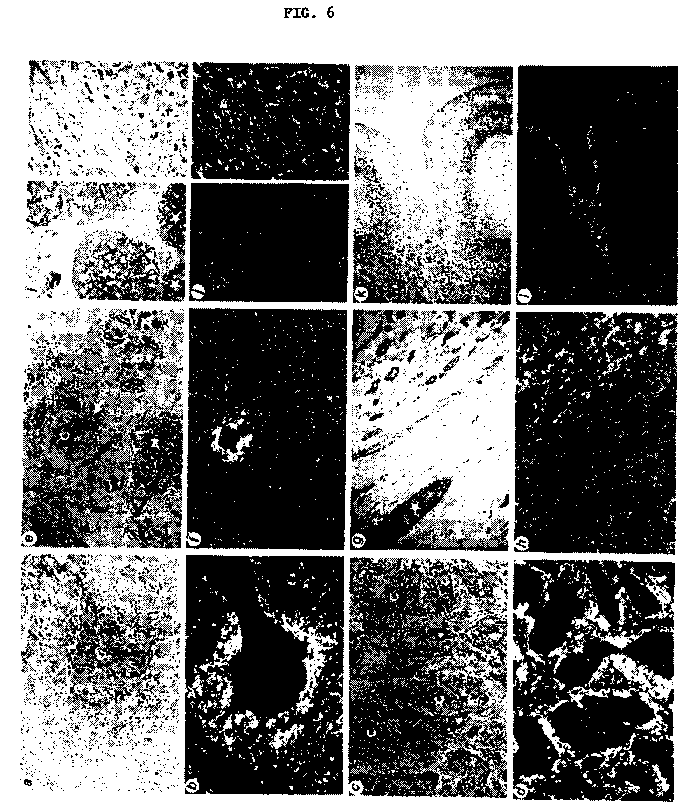

- Figure 6: Localisation des produits de transcription d'ARN de stromélysine-3 dans des sections de carcinome du sein et de bourgeons de membres embryonnaires
Micrographies à fond clair de sections de tissus (X 100) colorées à l'hématoxyline (A, C, E, G, I et K) ; et images à fond noir des mêmes sections (encore colorées à l'hématoxyline) après hybridation in situ avec de l'ARNc de stromélysine-3 anti-sens (B, D, F, H, J, et L) comme décrit à l'Exemple 6.

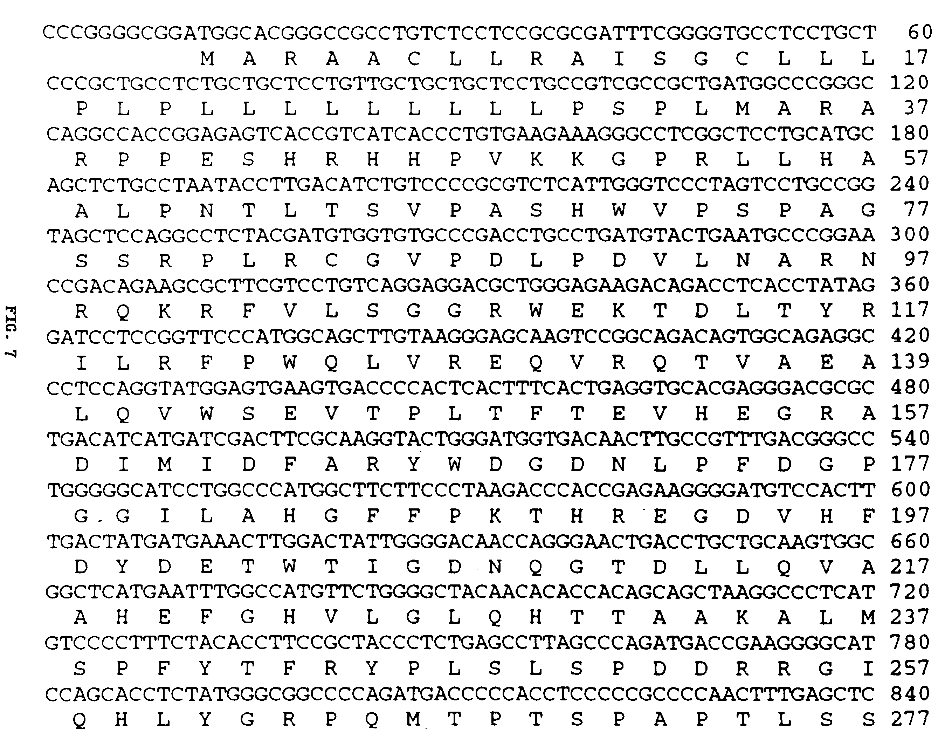

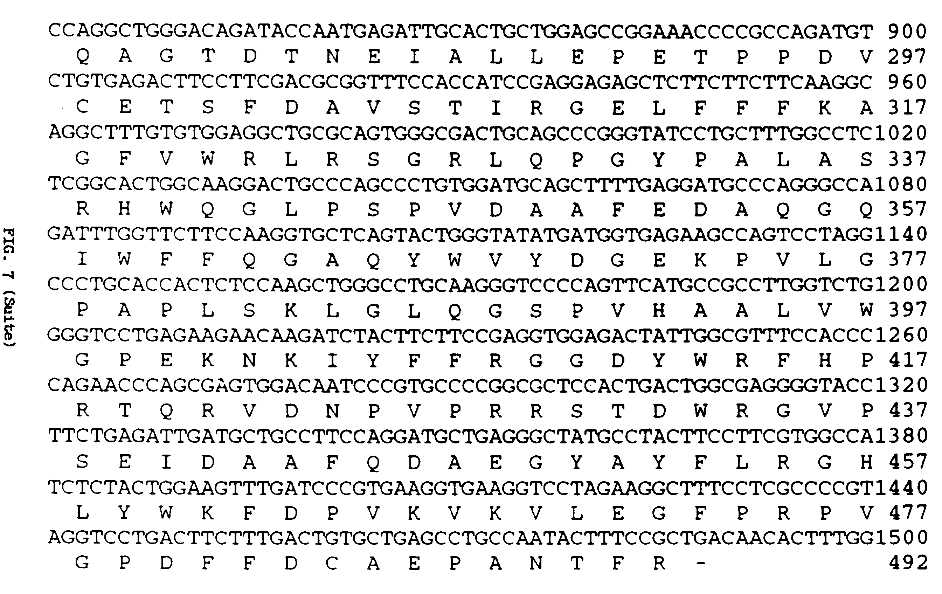

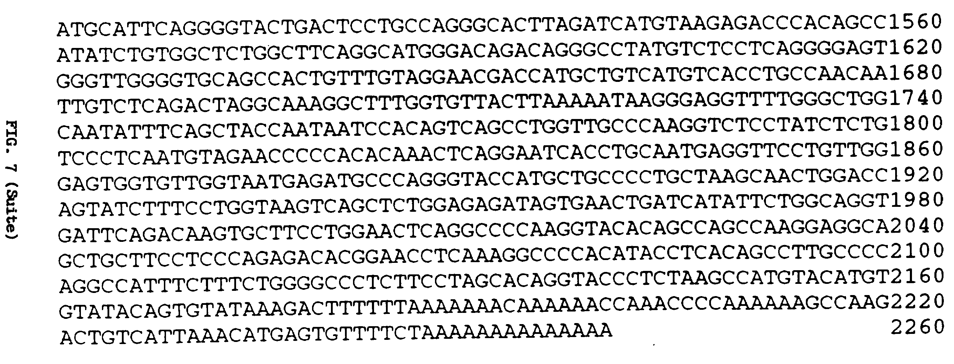

- Figure 7: Séquence d'ADNc de l'ADNc de ST3 de souris
La séquence nucléotidique de l'ADNc et la séquence d'acides aminés déduite de la ST3 de souris.

Sous un premier aspect, la présente invention propose un procédé pour le diagnostic du cancer invasif, en particulier du sein, et des carcinomes de la tête et du cou, et de la peau, comprenant la détection soit de stromélysine-3, soit d'une séquence nucléotidique codant pour la stromélysine-3.

Sous un autre aspect, la présente invention propose l'utilisation d'un agent pour perturber la synthèse ou l'activité de la stromélysine-3 dans le traitement ou la prophylaxie d'un cancer invasif, en particulier du sein, et des carcinomes de la tête et du cou, et de la peau.

On comprendra que les tumeurs métastatiques sont invasives, mais que les tumeurs invasives ne sont pas nécessairement métastatiques (par exemple, carcinomes de la peau de type baso-cellulaire.

Etant donné que l'expression du gène de la stromélysine-3 est spécifique des régions de dégradation de la MEC et code apparemment pour une métalloprotéinase, il est supposé que son activité de dégradation de la MEC est primordiale pour l'évolution de la tumeur en métastases. L'expression de la stromélysine-3 par les cellules du stroma est susceptible de détériorer une partie importante de la MEC, permettant ainsi aux cellules cancéreuses de migrer hors de la tumeur parentale.

En conséquence, tout agent qui peut affecter l'activité de la stromélysine-3 aura un effet sur les métastases. De tels agents seront de façon appropriée ceux, qui ou bien empêcheront la synthèse de la protéine, empêcheront la maturation de la protéine, ou bien modifieront l'activité de l'enzyme, soit par blocage, soit par modification de son activité.

L'expression du gène de la stromélysine-3 a été trouvée comme étant, dans le premier cas, capable de permettre le diagnostic du cancer du sein dans la phase métastatique. En fait, ce résultat a été obtenu par la détection de l'ARNm dans une diversité de tumeurs resectionnées. Le cancer du sein a été choisi, étant donné qu'il est responsable du taux de mortalité le plus élevé par cancer, dans la population des femmes non-fumeuses.

La stromélysine-3 est une nouvelle protéine appartenant presque certainement à la famille des MMPs, et elle est associée aux carcinomes du sein invasifs indépendamment de leur état hormonal.

Les membres de la famille des MMPs nécessitent une étape d'activation, qui peut être associée à l'élimination des pré- et des pro-séquences, pour devenir actifs. La séquence des acides aminés de la pro-stromélysine-3 et de la stromélysine-3 mature est notablement différente de celle des MMPs préalablement caractérisées, et peut manifester des propriétés distinctes concernant la maturation, l'activation et la spécificité pour les composants de la MEC.

Le gène de la stromélysine-3 est exprimé par tous les carcinomes du sein invasifs primaires, par certaines de leurs métastases, et dans des tissus dans lesquels on sait qu'un remodelage de la MEC extensif se produit (utérus, placenta, et bourgeons de membres), analysés pour une telle expression, mais non dans les fibroadénomes du sein et les tissus adultes normaux, ce qui suggére que le produit du géne de la stromélysine-3 joue un rôle important dans l'évolution du cancer du sein. Egalement en accord avec ce concept, le gène de la stromélysine-3 n'est pas exprimé dans la plupart des carcinomes du sein in situ, à l'exception des carcinomes in situ du type comédo, qui sont habituellement considérés comme des lésions préinvasives et sont souvent associés à une micro-invasion. Ainsi, la présence de produits de transcription de l'ARN de la stromélysine-3 dans des concentrations autres que les faibles concentrations trouvées partout ailleurs dans le corps, excepté dans l'utérus ou le placenta, permet de diagnostiquer un cancer métastatique ou un cancer ayant un risque élevé de devenir invasif.

La stromélysine-3 peut être mise en jeu dans les processus lytiques qui sont susceptibles d'être associés avec une croissance tumorale invasive. En variante, il est possible que la stromélysine-3 puisse également jouer un rôle dans la formation de la desmoplasie, laquelle est associée avec la plupart des lésions du cancer du sein invasif et peut représenter une réaction hôte pour empêcher un nouvel étalement des cellules malignes (Ahmed, A., Pathol. Annu. 25 (Pt2):237-286 (1990)). Dans un tel cas, l'augmentation de l'activité de stromélysine-3 serait avantageuse.

De plus, l'expression limitée du gène de la stromélysine-3 dans les fibroblastes du stroma entourant immédiatement les îlots cellulaires néoplasiques est remarquablement en contraste avec la collagénase IV, une autre métalloprotéinase connue pour être associée avec la conversion maligne de certaines cellules tumorigènes, et la cathépsine D, une aspartyl protéase lysosomale dont l'expression est accrue dans les carcinomes du sein, les deux étant exprimées, non dans les fibroblastes, mais dans les cellules épithéliales néoplasiques des cancers du sein (Monteagudo et al., Am. J. Pathol. 136:585-592 (1990) ; Garcia et al., Steroid Biochem. 27:439-445 (1987)).

Pour identifier le nouveau marqueur du cancer du sein, une banque d'ADNc a été élaborée, et de laquelle on a extrait l'ARN poly(A+) provenant d'une source de fibroadénome. Par ce procédé, la banque d'ADNc a été enrichie pour obtenir des séquences caractéristiques des cancers métastatiques.

Un certain nombre de clones ont été développés et triés à l'aide de sondes issues de l'ARN poly(A⁺) provenant de tumeurs métastatiques et de fibroadénomes. Les clones qui se lient plus fortement aux sondes issues de l'ARN poly(A⁺) de cancer métastatique ont ensuite encore été développés.

Parmi les clones produits de cette manière, l'un a été découvert comme étant exprimé de façon différentielle dans la mesure où des taux élevés d'expression ont été trouvés seulement dans les cancers malins du sein et du pharynx, les carcinomes de la tête, du cou et de la peau (du type cellules squameuses et du type cellules basales), de même que dans l'utérus et le placenta, dans la totalité desquels il y a une détérioration de la MEC, lequel, lorsqu'il est associé à un cancer, permet aux cellules cancéreuses de se propager dans tout le corps (métastases).

Dans le cas de l'utérus et du placenta, la détérioration de la MEC se produit naturellement, alors que le même événement partout ailleurs est susceptible d'être caractéristique d'une croissance tumorale.

Il est également intéressant de noter que l'expression du gène de la stromélysine-3 a été trouvée dans la différenciation interdigitale pendant la formation des bourgeons de membres dans le foetus, qui est associée à la détérioration de la MEC.

Une caractérisation de la séquence d'ADNc a montré qu'il y avait un cadre de lecture ouvert. Une comparaison de la séquence protéinique codée avec une banque connue a établi que la protéine appartenait à une famille connue pour détériorer la MEC. Bien que la séquence de la stromélysine-3 présente moins d'analogie avec les autres membres de sa famille que n'importe lesquels des autres membres présentent les uns pour les autres, elle présente néanmoins un certain nombre de régions caractéristiques qui servent à identifier la nature de l'enzyme. En conséquence, la protéine a été appelée stromélysine-3, bien qu'elle puisse être une collagénase, ou puisse détériorer un constituant différent de la MEC en même temps.

La construction de sondes nucléotidiques pour établir la présence de l'ARNm de la stromélysine-3 a révélé une distribution dans les tissus telle que décrite ci-dessus, et a permis également à des photomicrographies de localiser exactement les zones d'expression du gène de la stromélysine-3 par marquage.

Une analyse des photomicrographies produites par ce procédé a montré, de façon quelque peu surprenante, que le gène de la stromélysine-3 n'était pas exprimé par les cellules cancéreuses elles-mêmes, mais dans le stroma environnant. De plus, le stroma ne s'est pas avéré présenter d'ARNm de la stromélysine-3, lorsque la membrane basale de la tumeur était encore intacte (voir Figure 6). Le gène de la stromélysine-3 est exprimé par tous les carcinomes du sein invasifs primaires, par certains de leurs noeuds métastatiques, et dans des tissus dans lesquels un remodelage de la MEC extensif est connu comme se produisant (utérus, placenta et bourgeons de membres), analysés pour une telle expression, mais non dans les fibroadénomes du sein et les tissus adultes normaux, suggérant que le produit du gène de la stromélysine-3 joue un rôle important dans l'évolution du cancer du sein. Egalement en accord avec ce concept, le gène de la stromélysine-3 n'est pas exprimé dans la plupart des carcinomes du sein in situ, à l'exception des carcinomes in situ du type comédo, qui sont habituellement considérés comme des lésions préinvasives et sont souvent associés à une micro-invasion. La stromélysine-3 est toujours présente dans le stroma de cancers métastatiques, mais pas dans le stroma des tumeurs primaires in situ (des tumeurs ayant encore une membrane basale et qui sont non-invasives). Ainsi, la présence des produits de transcription de l'ARN de la stromélysine-3 dans des concentrations autres que les faibles concentrations trouvées partout ailleurs dans le corps, excepté dans l'utérus et le placenta, permet de diagnostiquer un cancer métastatique ou un cancer ayant un risque élevé de devenir invasif.

De plus, l'expression du gène de la stromélysine-3 n'a pas été détecté dans toutes les lignées cellulaires de cancer du sein positif ou négatif aux RO (Récepteurs d'Oestrogènes), même si certaines d'entre elles sont connues pour sécréter et posséder des récepteurs pour EGF/TGF-α et FGF (facteurs qui sont impliqués dans l'expression du gène de la stromélysine-3).

En conséquence, les techniques de détection standards appliquées à la stromélysine-3, ses précurseurs ou ses séquences nucléotidiques codantes, peuvent être utilisées pour diagnostiquer un cancer métastatique, ou pour confirmer qu'une tumeur primaire n'a pas encore atteint la phase métastatique mortelle.

De telles techniques peuvent comprendre la détection par des sondes nucléotidiques, de la manière telle que celle décrite ci-dessus, ou bien peuvent comprendre la détection de la protéine stromélysine-3, par exemple, par des anticorps ou leurs équivalents.

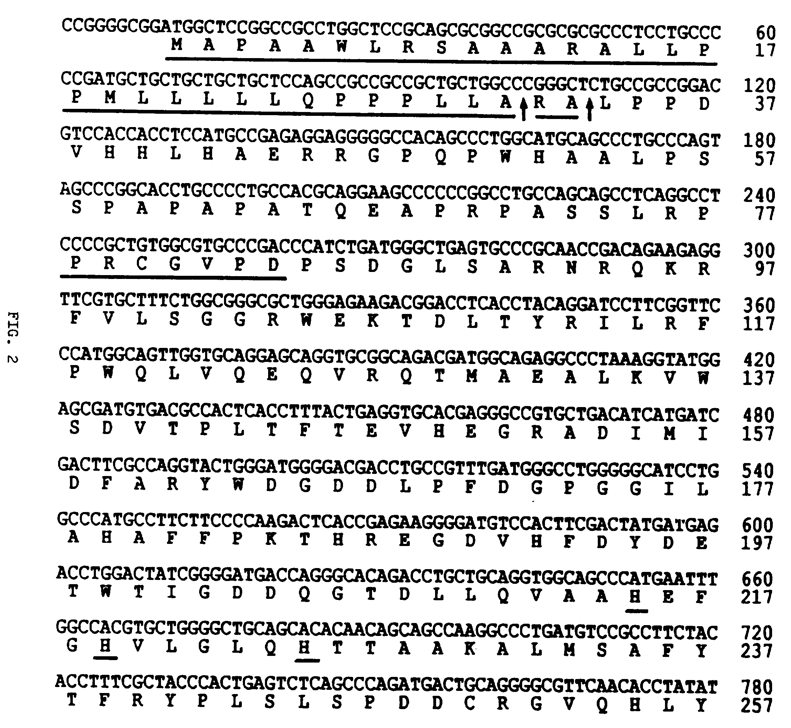

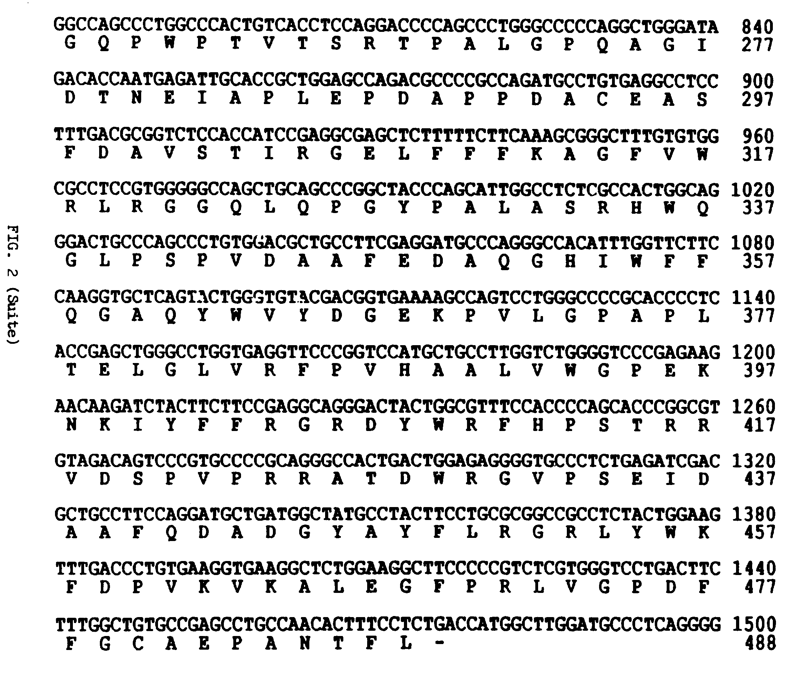

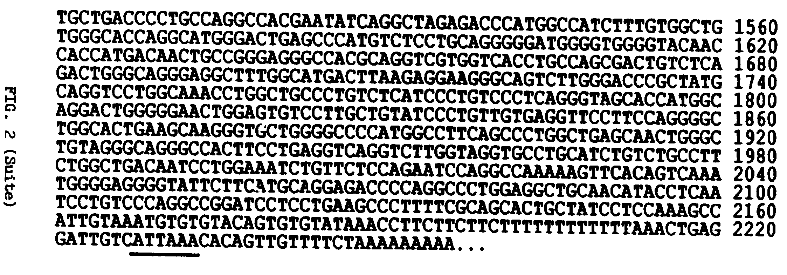

Les sondes nucléotidiques peuvent être n'importe quelles sondes qui s'hybrideront plus fortement avec la séquence représentée sur la Figure 2 annexée qu'avec les autres séquences existant naturellement. Des types de sondes comprennent l'ADNc, les ribosondes, les oligonucléotides de synthèse et les sondes génomiques. Le type de sonde utilisé sera généralement imposé par la situation particulière, tel que des ribosondes pour une hybridation in situ, et l'ADNc pour l'analyse Northern blot, par exemple. Les sondes que l'on préfère le plus sont celles qui correspondent au brin négatif de l'ADNc de la Figure 2. Il est également possible de mettre en oeuvre des sondes qui reconnaissent des introns localisés à l'intérieur du gène de la stromélysine-3, mais ceci n'est pas nécessairement aussi fiable que la détection des produits de transcription de l'ARN.

La détection du gène codant pour la stromélysine-3, en tant que tel, n'est généralement d'aucune utilité dans le diagnostic, mais d'autres formes d'essai pour détecter les produits de transcription et autres produits d'expression seront généralement utiles. Les sondes peuvent être aussi courtes que cela est nécessaire pour reconnaître de façon différentielle les produits de transcription de l'ARNm de la stromélysine-3, et peuvent être aussi courtes que, par exemple, 15 nucléotides.

La forme de marquage des sondes peut être n'importe quelle forme qui est appropriée, telle que l'utilisation de radioisotopes, par exemple ³²P et ³⁵S. Le marquage par des radioisotopes peut être obtenu, que la sonde soit synthétisée chimiquement ou biologiquement, par l'utilisation de bases marquées de façon appropriée. D'autres formes de marquage peuvent comprendre le marquage par enzyme ou par anticorps, tel que cela est caractéristique de l'ELISA, mais la détection des produits de transcription de l'ARNm par des sondes marquées se fera généralement par voie de radiographie X.

La détection de produits de transcription de l'ARN peut être obtenue par l'analyse par Northern blot, par exemple, dans laquelle une préparation d'ARN est posée sur un gel d'agarose dénaturant, et transférée sur un support approprié, tel que de la cellulose activée, de la nitrocellulose activée ou du verre ou des membranes de Nylon. L'ADNc ou l'ARN radiomarqué est ensuite hybridé avec la préparation, lavé et analysé par autoradiographie.

Une visualisation d'hybridation in situ peut également être employée (Exemple 6), dans laquelle une sonde d'ARNc anti-sens, marquée par [³⁵S], est hybridée avec une section fine d'un échantillon de biopsie, lavée, clivée par de la RNase et exposée à une émulsion sensible pour l'autoradiographie. Les échantillons peuvent être colorés à l'hématoxyline pour démontrer la composition histologique de l'échantillon, et une micrographie à fond noir avec un filtre de lumière approprié fait ressortir l'émulsion développée.

L'immunohistochimie peut être utilisée pour détecter l'expression de stromélysine-3 dans un échantillon de biopsie. Un anticorps approprié est amené en contact, par exemple, avec une couche mince de cellules, lavé, puis mis en contact avec un second anticorps marqué. Le marquage peut être fait par une enzyme, telle que la peroxydase, l'avidine, ou par radiomarquage. Des marqueurs chromogènes sont généralement préférables, étant donné qu'ils peuvent être détectés au microscope.

D'une manière plus générale, on préfère détecter la protéine par essai immunologique, par exemple, par ELISA ou RIA, qui peut être extrêmement rapide. Ainsi, on préfère généralement utiliser des anticorps, ou des équivalents d'anticorps, pour détecter la stromélysine-3, mais l'utilisation d'un substrat de stromélysine-3 marqué d'une manière appropriée peut être également avantageuse.

Il peut ne pas être nécessaire de marquer le substrat, à la condition que le produit du processus enzymatique soit détectable et caractéristique en soi (tel que le peroxyde d'hydrogène par exemple). Cependant, s'il est nécessaire de marquer le substrat, ceci peut alors comprendre le marquage enzymatique, le marquage par radioisotopes, le marquage par anticorps, le marquage par marqueur fluorescent ou toute autre forme appropriée qui apparaîtra aisément à l'évidence à l'homme du métier.

Ce que l'on préfère le plus pour la détection de l'expression de stromélysine-3 est l'utilisation d'anticorps. Les anticorps peuvent être préparés comme décrit ci-après, et utilisés de n'importe quelle manière appropriée pour détecter l'expression de stromélysine-3.

Des techniques utilisant les anticorps comprennent ELISA (essai par immunosorbant lié à une enzyme - enzyme linked immunosorbent assay) et RIA (essai radioimmunologique - radioimmunoassay). Tout mode opératoire classique peut être employé pour de tels essais immunologiques. Les modes opératoires peuvent être conduits d'une manière appropriée de telle sorte que : une stromélysine-3 standard est marquée par un radioisotope tel que ¹²⁵I ou ³⁵S, ou une enzyme titrable, telle que la peroxydase de raifort ou la phosphatase alcaline et, conjointement avec l'échantillon non-marqué, elle est amenée en contact avec l'anticorps correspondant, sur quoi un second anticorps est utilisé pour lier le premier et la radio-activité ou l'enzyme immobilisée est analysée (essai par compétition) ; en variante, on fait réagir la stromélysine-3 dans l'échantillon avec l'anticorps immobilisé correspondant, on fait réagir avec le système l'anticorps anti-stromé-lysine-3 marqué par un radioisotope ou une enzyme, et on dose la radioactivité ou l'enzyme (essai ELISA - en sandwich). D'autres méthodes classiques peuvent être également employées si cela est approprié.

Les techniques ci-dessus peuvent être conduites essentiellement sous la forme d'un essai "à une seule étape" ou "à deux étapes". L'essai "à une seule étape" implique la mise en contact de l'antigène avec l'anticorps immobilisé et, sans lavage, la mise en contact du mélange avec l'anticorps marqué. L'essai "à deux étapes" implique un lavage avant la mise en contact du mélange avec l'anticorps marqué. D'autres méthodes classiques peuvent être également employées si cela est approprié.

Le marquage enzymatique et le radio-marquage de la stromélysine-3 et/ou des anticorps peuvent être effectués par des moyens classiques. De tels moyens comprendront généralement la liaison covalente de l'enzyme à l'antigène ou à l'anti-corps en question, tel que par le glutaraldéhyde, spécifiquement pour ne pas affecter de façon défavorable l'activité de l'enzyme, ce par quoi on entend que l'enzyme doit rester capable d'interagir avec son substrat, bien qu'il ne soit pas nécessaire que la totalité de l'enzyme soit active, suffisamment d'enzyme active devant subsister pour permettre à l'essai d'être effectué. Naturellement, certaines techniques pour lier l'enzyme sont non-spécifiques (telles que l'utilisation de formaldéhyde), et conduiront seulement à une partie d'enzyme active.

Il est habituellement souhaitable d'immobiliser un composant du système d'essai sur un support, ce qui permet aux autres composants du système d'être amenés en contact avec le composant et d'être facilement retirés sans opération laborieuse et prenant du temps. Il est possible qu'une deuxième phase soit immobilisée à distance de la première, mais une seule phase est habituellement suffisante.

Il est possible d'immobiliser l'enzyme elle-même sur un support, mais si une enzyme en phase solide est requise, ceci est alors généralement obtenu au mieux par liaison à l'anticorps et fixation de l'anticorps à un support, pour lequel des modèles et des systèmes sont bien connus dans la technique. Un simple polyéthylène peut fournir un support approprié.

Les enzymes que l'on peut employer pour le marquage ne sont pas limitées de façon particulière, mais peuvent être choisies parmi les éléments du groupe des oxydases, par exemple. Celles-ci catalysent la production de peroxyde d'hydrogène par réaction avec leurs substrats, et la glucose oxydase est souvent utilisée pour sa bonne stabilité, la facilité avec laquelle on peut se la procurer, et son bas prix, ainsi que la disponibilité aisée de son substrat (glucose). L'activité de l'oxydase peut être déterminée par mesure de la concentration de peroxyde d'hydrogène formé après réaction de l'anticorps marqué par l'enzyme avec le substrat dans des conditions contrôlées bien connues dans la technique.

D'autres techniques peuvent être utilisées pour détecter la stromélysine-3 en suivant les préférences. Une technique de ce type est l'analyse Western blot (Towbin et al., Proc. Nat. Acad. Sci. 76:4350 (1979)), dans laquelle un échantillon traité de manière appropriée est déposé sur un gel de SDS-PAGE avant d'être transféré sur un support solide, tel qu'un filtre de nitrocellulose. Des anticorps anti-stromélysine-3 (non-marqués) sont ensuite amenés en contact avec le support et testés par un réactif immuno-logique secondaire, tel que la protéine A marquée ou l'anti-immunoglobuline marquée (des marqueurs appropriés incluant ¹²⁵I, la peroxyde de raifort et la phosphatase alcaline).

Des échantillons pour les besoins de diagnostic peuvent être obtenus à partir de n'importe quel site approprié. Un échantillon obtenu directement à partir de la tumeur, tel que le stroma ou le cytosol, peut être idéal, mais il peut être également approprié d'obtenir l'échantillon à partir du sang, par exemple. Cependant, si l'échantillon est issu du sang, des essais très sensibles peuvent être requis, étant donné que la quantité de stromélysine-3 serait alors diluée dans le flux sanguin. Un tel diagnostic peut être d'importance particulière pour surveiller l'évolution d'un patient, par exemple, après une opération chirurgicale pour éliminer une tumeur. Si une lecture de référence est faite après l'opération, puis d'autres, faites à intervalles réguliers, toute augmentation pourrait être indicatrice d'une rechute, ou éventuellement d'une métastase. Effectuer de telles lectures peut nécessiter de prendre en considération l'activité dans l'utérus, par exemple.

Des anticorps anti-stromélysine-3 peuvent être également utilisés pour des besoins d'imagerie. En dehors des enzymes, d'autres marqueurs appropriés comprennent les radioisotopes, l'iode (¹²⁵I, ¹²¹I), le carbone (¹⁴C), le soufre (³⁵S), le tritium (³H), l'indium (¹¹²In), et le technétium (^{99m}TC), des marqueurs fluorescents, tels que la fluorescéine et la rhodamine, et la biotine.

Cependant, pour des besoins d'imagerie in vivo, la situation devient plus restrictive, étant donné que les anticorps ne sont pas détectables, en tant que tels, de l'extérieur du corps, et doivent donc être marqués, ou autrement modifiés, pour permettre la détection.

Des marqueurs pour cet objectif peuvent être n'importe quels marqueurs qui ne gênent pas de façon substantielle la liaison de l'anticorps, mais qui permettent une détection extérieure. Des marqueurs appropriés peuvent comprendre ceux qui peuvent être détectés par radiographie X, RMN ou RSE (Résonance de Spin Electronique). Pour les techniques de radiographie X, des marqueurs appropriés comprennent tout radioisotope qui émet un rayonnement détectable mais qui n'est pas manifestement nuisible pour le patient, tel que le baryum ou le césium, par exemple. Des marqueurs appropriés pour RMN et RSE comprennent généralement ceux ayant un spin caractéristique détectable, tel que le deutérium, qui peuvent être incorporés dans l'anticorps par marquage approprié de nutriments pour l'hybridome concerné, par exemple.

Dans le cas des méthodes d'imagerie in vivo, un anticorps ou un fragment d'anticorps, qui a été marqué par une fraction pour imagerie détectable appropriée, tel qu'un radioisotope (par exemple, ¹³¹I, ¹¹²In, ^{99m}Tc), une substance radio-opaque, ou une matière détectable par résonance magnétique nucléaire, est introduit (par exemple, par voie parentérale, sous-cutanée ou intrapéritonéale) dans le sujet, (tel qu'un sujet humain) devant être examiné.

La dimension du sujet, et le système d'imagerie utilisé, détermineront la quantité de fractions pour imagerie requise pour produire des images de diagnostic. Dans le cas d'une fraction de radioisotope, pour un sujet humain, la quantité de radioactivité injectée se situera normalement dans la plage d'environ 5 à 20 millicuries de technétium 99m. L'anticorps ou le fragment d'anticorps marqué s'accumulera ensuite de préférence à l'emplacement des cellules qui contiennent la stromélysine-3. L'anticorps ou le fragment d'anticorps marqué peut être ensuite détecté à l'aide de techniques connues.

Pour une discussion générale de ce domaine technologique, on se reportera à S.W. Burchiel et al., "Immunopharmacocinétique des Anticorps Radiomarqués et de Leurs Fragments (Immunopharmacokinetics of Radiolabelled Antibodies and Their Fragments)" (Chapitre 13 dans Imagerie des Tumeurs : La Détection Radiochimique du Cancer (Tumor Imaging : The Radiochemical Detection of Cancer), éds., S.W. Burchiel et B.A. Rhodes, Masson Publishing Inc. (1982)).

Les anticorps peuvent être dirigés soit contre un peptide de stromélysine-3, soit contre la totalité de la molécule. Un tel peptide peut être présenté conjointement avec une protéine support, telle qu'une albumine, à un système animal ou, s'il est suffisamment long, à savoir 25 restes d'acides aminés, sans support. Il est peu probable que des anticorps humains soient capables de reconnaître la stromélysine-3, étant donné que cette protéine représentera une auto-protéine.

Tel qu'utilisé ici, le terme "peptide" désigne toute molécule comprenant au moins 2 acides aminés liés par une liaison peptidique. En tant que tel, le terme inclut les oligopeptides, les polypeptides et les protéines.

Des anticorps polyclonaux obtenus par la technique ci-dessus peuvent être utilisés directement, ou des cellules productrices d'anticorps appropriées peuvent être isolées à partir de l'animal et utilisées pour former un hybridome par des moyens connus (Kohler et Milstein Nature 256:795 et suivantes (1975)). La sélection d'un hybridome approprié sera également évident pour l'homme du métier, et l'anticorps résultant peut être utilisé dans un essai approprié pour identifier la stromélysine-3.

Des anticorps, ou leurs équivalents, peuvent être également utilisés conformément à la présente invention pour le traitement ou la prophylaxie des cancers métastatiques. L'administration d'une dose appropriée de l'anticorps peut servir à inhiber la production, ou à inhiber l'activité effective de la stromélysine-3, et ceci peut fournir un espace de temps crucial dans lequel doit être traitée la croissance maligne.

La prophylaxie peut être appropriée même à des stades très précoces de la maladie, étant donné qu'on ne soit pas ce qui conduit réellement à la métastase dans n'importe quel cas donné. Ainsi, l'administration des anticorps, de leurs équivalents, ou de facteurs, tels que les TIMPs (composés existant naturellement qui régulent les MMPs - inhibiteurs tissulaires des métalloprotéinases), qui gênent l'activité de la stromélysine-3, peut être effectuée dès que le cancer est diagnostiqué, et le traitement poursuivi aussi longtemps que nécessaire, de préférence jusqu'à ce que la menace de la maladie ait été éliminée.

Une forme préférée de traitement consiste à employer la technique dite de "balle magique", dans laquelle une toxine appropriée est fixée aux anticorps qui ciblent ensuite la zone de la tumeur. De telles toxines sont bien connues dans la technique, et peuvent comprendre des radioisotopes toxiques, des métaux lourds, des enzymes et des activateurs du complément, ainsi que des toxines naturelles telles que la ricine, qui sont capables d'agir au niveau seulement d'une ou deux molécules par cellule. Il peut être également possible d'utiliser une technique qui consiste à délivrer des doses localisées d'antagonistes d'hormones ou tous autres composés physiologiquement actifs appropriés, qui peuvent être utilisées, par exemple, pour traiter les cancers.

On comprendra que des anticorps destinés à être utilisés conformément à la présente invention, que ce soit pour des applications de diagnostic ou thérapeutiques, puissent être monoclonaux ou polyclonaux, comme cela est approprié. Des équivalents d'anticorps de ceux-ci peuvent comprendre : les fragments Fab' des anticorps, tels que Fab, Fab', F(ab')₂ et Fv ; des idiotopes ; ou les produits résultant du greffage allotopique (où la région de reconnaissance d'un anticorps animal est greffée dans la région appropriée d'un anticorps humain pour éviter une réponse immune chez le patient), par exemple. D'autres modifications et/ou agents appropriés apparaîtront à l'évidence à l'homme du métier.

En plus de l'utilisation des anticorps pour inhiber ou éliminer la stromélysine-3, il peut être également possible d'utiliser d'autres formes d'inhibiteur. De tels inhibiteurs peuvent être généraux (pour les enzymes de dégradation de la MEC, par exemple), ou spécifiques de la stromélysine-3. On sait qu'il existe des inhibiteurs tissulaires des métalloprotéinases (TIMPs), et il est extrêmement probable qu'il y ait un TIMP spécifique pour la stromélysine-3. Un tel TIMP est facilement identifiable par des techniques standards.

Des inhibiteurs de synthèse de la stromélysine-3 peuvent être également fabriqués, et ceux-ci correspondront généralement à la zone du substrat affectée par l'activité enzymatique. On préfère généralement que de tels inhibiteurs correspondent à un intermédiaire fixé entre le substrat et les produits de clivage, mais il est également possible de fournir une version stériquement encombrée du site de liaison, ou une version du site de liaison qui sera elle-même liée d'une manière irréversible à la stromélysine-3. D'autres inhibiteurs appropriés apparaîtront à l'évidence à l'homme du métier.

D'autres méthodes pour bloquer l'activité de la stromélysine-3 peuvent être également employées. Celles-ci peuvent consister en des agents dénaturants, par exemple, bien que ceux-ci aient tendance à être non-spécifiques et pourraient être employés d'une manière appropriée seulement s'ils pouvaient être ciblés, par exemple par l'utilisation d'anticorps spécifiques. D'autres formes d'activité de blocage de la stromélysine-3 pourraient consister en un blocage de l'évolution depuis une pré-proprotéine jusqu'à une protéine. Ce procédé fournit plusieurs étapes cibles, et il est seulement nécessaire d'identifier une étape qui peut être bloquée d'une manière indépendante de façon à ne pas affecter d'autres enzymes vitales, ou qui peut être à nouveau ciblée.

Il peut être également possible d'utiliser des peptides ou d'autres petites molécules pour reconnaître d'une manière sélective une structure tertiaire sur la stromélysine-3, bloquant ainsi son activité enzymatique. Un tel agent de blocage d'activité n'a pas besoin nécessairement d'être lié au site actif, mais peut servir à modifier ou fixer la structure tertiaire de la stromélysine-3, en détruisant, mettant en suspens ou modifiant son activité. L'agent de blocage n'a pas besoin également d'agir nécessairement par lui-même, mais peut être lié à une autre molécule pour cet objectif, ou peut servir comme site de reconnaissance pour un agent d'inactivation approprié.

Nos études ont démontré que la présence d'ARNm de collagénase de type I et d'ARN de collagénase de type IV de 92 kD est exclusivement associée aux tumeurs malignes, bien que l'inverse ne soit pas toujours vrai (autrement dit, les tumeurs ne sont pas toujours associées à ces protéines).

Il y a apparemment un parallèle entre l'expression du gène de la stromélysine-3 et celle du gène de la ténascine dans les carcinomes du sein invasifs. La glycoprotéine de la MEC qu'est la ténascine (Chiquet-Ehrismann et al. Cell 47:131-139 (1986)) apparaît jouer un rôle essentiel dans les interactions des cellules épithéliales et des cellules mésenchymateuses et la migration cellulaire pendant le développement normal, incluant celle de la glande mammaire pendant l'organogenèse.

On a trouvé que la ténascine était logiquement surexprimée dans le stroma fibreux des tumeurs malignes du sein, et apparaît être induite d'une manière analogue à la stromélysine-3. Lorsqu'elle est comparée à la fibronectine, la ténascine est un substrat médiocre pour la fixation des cellules épithéliales de tumeurs mammaires, suggérant qu'elle peut leur permettre de devenir invasives.

Ainsi, la stromélysine-3 peut agir de concert avec la ténascine pendant la phase d'invasion du cancer du sein. La stromélysine-3 et la ténascine peuvent être également co-exprimées pendant l'embryogenèse dans les régions dans lesquelles les interactions épithélium-mésenchyme sont connues comme jouant un rôle important, et dans lesquelles se produit la migration cellulaire.

Par conséquent, la présente invention propose également un procédé pour le diagnostic du cancer métastatique tel que défini ci-dessus, comprenant en outre la détection de l'une quelconque des protéines précédentes, ou d'une séquence nucléotidique codant pour elles.

L'invention propose également une utilisation dans le traitement ou la prophylaxie du cancer métastatique, comprenant en outre l'utilisation d'un agent pour lier l'une quelconque des protéines précédentes.

La présente invention propose en outre une séquence nucléotidique codant pour la totalité ou une partie qui est unique pour la stromélysine-3. La séquence de stromélysine-3 est, de préférence, celle représentée sur la Figure 2 des dessins annexés, et la séquence nucléotidique est également, de préférence, celle représentée sur la Figure 2. Cependant, on comprendra que la séquence nucléotidique puisse être substantiellement différente de celle représentée sur la Figure, en raison de la dégénérescence dans le code génétique, à condition qu'elle code toujours pour au moins une partie qui est unique pour la stromélysine-3.

La séquence nécessaire peut encore varier davantage, selon l'utilisation à laquelle elle est destinée. Si elle est destinée à être utilisée pour détecter des produits de transcription de l'ARN dans des échantillons biologiques, il sera alors habituellement préférable qu'elle corresponde de façon plus proche à la séquence donnée sur la Figure 2. Cependant, la séquence peut varier encore, à condition que l'hybridation soit possible dans les conditions de rigueur choisies.

Une sonde peut être obtenue par reverse-engineering par l'homme du métier à partir de la séquence peptidique de la Figure 2. Cependant l'utilisation de telles sondes peut être limitée, car on comprendra que toute séquence donnée obtenue par reverse-engineering ne s'hybridera pas nécessairement bien, ou ne s'hybridera pas du tout, avec toute séquence complémentaire donnée obtenue par reverse-engineering à partir du même peptide, en raison de la dégénérescence du code génétique. Ceci est un facteur courant dans les calculs de l'homme du métier, et la dégénérescence de toute séquence donnée est fréquemment si vaste qu'elle conduit à un nombre important de sondes pour n'importe quelle séquence.

Si la séquence nucléotidique est requise pour l'expression d'un peptide de stromélysine-3 ou de l'enzyme entière, il peut alors y avoir une dérive considérablement plus importante, à la fois comme décrit ci-dessus en ce qui concerne le code génétique, et également en ce qui concerne le fait que certaines séquences d'acides aminés de la stromélysine-3 peuvent être amenées à varier sans effet significatif sur la structure ou la fonction de l'enzyme.

Si de telles différences de séquences sont envisagées, on doit alors garder à l'esprit qu'il y aura des zones critiques sur la molécule qui déterminent l'activité. De telles zones comprendront habituellement des restes qui constituent le site de liaison, ou qui forment des structures tertiaires qui affectent le site de liaison. En général, il est possible de remplacer des restes qui forment la structure tertiaire, à condition que des restes jouant une fonction analogue soient utilisés. Dans d'autres cas, le type de restes peut être complètement sans importance.

Par conséquent, la présente invention comprend également tous variants et mutants sur la séquence qui présentent encore une activité stromélysine-3 substantielle, ou qui présentent des régions caractéristiques de la stromélysine-3, pour une utilisation dans la fabrication d'anticorps, par exemple. De tels variants et mutants comprennent des délétions, des additions, des insertions, des inversions, des répétitions et de substitutions de type (par exemple, substitution d'un reste hydrophile par un autre, mais en principe pas de substitution d'un reste fortement hydrophobe par un résidu fortement hydrophile). De petites modifications auront généralement un faible effet sur l'activité, à moins qu'elles soient une partie essentielle de la molécule, et peuvent être un produit secondaire de manipulation génétique, par exemple, lors de la production de sites de restriction supplémentaires, si ceci est désiré. Une modification peut également comprendre la substitution d'un ou plusieurs des restes par tout autre reste approprié, et une telle substitution peut être de 1:1 ou dans tout autre rapport approprié, supérieur ou inférieur à l'unité.

Des mutations de lieu et autres modifications dans la séquence codante peuvent être effectuées pour ajouter ou déléter des sites de restriction, par exemple, pour prêter autrement son assistance dans la manipulation/expression génétique, ou pour augmenter ou modifier autrement d'une manière appropriée la molécule de stromélysine-3.

On comprendra également qu'un équivalent de stromélysine-3 sera trouvé chez d'autres animaux, en particulier les mammifères, et que l'information des séquences à partir de telles sources peut être d'une importance particulière pour élucider les régions conservées de la molécule de stromélysine-3. Par exemple, la séquence correspondante chez la souris est conservée à - 80%, incluant une séquence telle que la séquence de 10 acides aminés dans le prodomaine caractéristique de la stromélysine-3. On comprendra également que des séquences animales correspondant aux séquences humaines de stromélysine-3 seront aisément détectables par des méthodes connues dans la technique et décrites ci-dessus, et que de telles séquences et leurs peptides, ainsi que les mutants et les variants de celles-ci, font partie de l'invention.

Les séquences de l'invention peuvent être également traitées par génie génétique pour fournir des sites de restriction, si on le souhaite. Ceci peut être effectué de façon à ce qu'il n'y ait pas interférence avec la séquence peptidique de la stromélysine-3 qui est codée, ou qu'il y ait dans n'importe quelle mesure souhaitée ou nécessaire, à la condition que le produit final ait les propriétés désirées.

Comme mentionné ci-dessus, bien que l'hybridation puisse être une indication peu sûre de l'homologie de séquences, des séquences préférées seront généralement celles présentant au-delà de 50%, de préférence, de 70% et, de façon davantage préférée, de 80% d'homologie avec la séquence de la Figure 2.

Comme avec les autres métalloprotéinases, la stromélysine-3 est initialement exprimée sous la forme d'une pré-proenzyme. Ainsi, deux étapes de clivage sont observées in vivo. Le clivage n'est pas nécessairement une exigence pour l'expression in vitro, et il peut être possible qu'E. coli, par exemple, exprime la protéine mature.

Lorsque l'on souhaite exprimer la stromélysine-3 ou un peptide caractéristique de celle-ci, n'importe quel système approprié peut être utilisé. La nature générale des vecteurs appropriés, des vecteurs d'expression et des produits d'assemblage pour ceux-ci apparaîtra à l'évidence à l'homme du métier.

Par "caractéristique", on entend tout peptide qui a une séquence unique pour la stromélysine-3. Une telle séquence peut être importante pour l'activité stromélysine-3, ou peut juste être une séquence non trouvée dans d'autre peptides. Cependant, des séquences importantes pour l'activité stromélysine-3 sont généralement préférées, étant donné que celles-ci sont davantage susceptibles d'être conservées à l'intérieur d'une population.

Des vecteurs d'expression appropriés peuvent être basés sur des phages ou des plasmides, lesquels sont tous deux généralement spécifiques de l'hôte, bien que ceux-ci puissent souvent être traités par génie génétique pour d'autres hôtes. D'autres vecteurs appropriés comprennent des cosmides et des rétrovirus, et tous autres véhicules, qui peuvent être ou ne pas être spécifiques d'un système donné. A nouveau, des séquences de contrôle, telles que des séquences de reconnaissance, des promoteurs, des opérateurs, des inducteurs, des terminateurs et d'autres séquences essentielles et/ou utiles dans la régulation de l'expression, apparaîtront aisément à l'homme du métier, et peuvent être associées à la séquence de stromélysine-3 naturelle ou au vecteur utilisé, ou peuvent être issues de n'importe quelle autre source comme cela est approprié. Les vecteurs peuvent être modifiés ou traités par génie génétique d'une manière appropriée.

Une préparation correcte des séquences nucléotidiques peut être confirmée, par exemple, par la méthode de Sanger et al. (Proc. Natl. Acad. Sci. USA 74:5463-7 (1977)).

Un fragment d'ADNc codant pour la stromélysine-3 de l'invention peut être facilement inséré dans un vecteur approprié. D'une manière idéale, le vecteur récepteur présente des sites de restriction appropriés pour la facilité de l'insertion, mais une soudure d'extrémités droites, par exemple, peut également être utilisée, bien que celle-ci puisse conduire à une incertitude sur le cadre de lecture et la direction d'insertion. Dans un tel cas, il est tout naturel de tester les transformants pour l'expression, dont 1 sur 6 doit avoir le cadre de lecture correct. Des vecteurs appropriés peuvent être choisis aisément par l'homme du métier en fonction du système d'expression désiré.

En transformant un organisme approprié ou, de préférence, une lignée cellulaire eucaryote, telle que HeLa, avec le plasmide obtenu, en choisissant le transformant avec de l'ampicilline ou par un autre moyen approprié si nécessaire, et en ajoutant du tryptophane ou un autre promoteur-inducteur approprié (tel que l'acide indole-acrylique) si nécessaire, on peut exprimer la stromélysine-3 désirée. L'étendue de l'expression peut être analysée par électrophorèse sur gel de SDS polyacrylamide - SDS-PAGE (Lemelli, Nature 227:680-685 (1970)).

Des méthodes appropriées pour faire croître et transformer des cultures, etc. sont illustrées utilement, par exemple, dans Maniatis (Molecular Cloning, A Laboratory Notebook, Maniatis et al. (éds.), Cold Spring Harbor Labs, NY (1989)).

Des cultures utiles pour la production de stromélysine-3, ou un peptide de celle-ci, peuvent être d'une manière appropriée des cultures de n'importe quelles cellules vivantes, et peuvent varier des systèmes d'expression procaryotes jusqu'aux systèmes d'expression eucaryotes. Un système procaryote préféré est celui d'E. coli, en raison de sa facilité de manipulation. Cependant, il est également possible d'utiliser un système supérieur, tel qu'une lignée cellulaire de mammifère, pour l'expression d'une protéine eucaryote. Des lignées cellulaires couramment préférées pour l'expression temporaire sont les lignées cellulaires HeLa et Cos. D'autres systèmes d'expression comprennent la lignée cellulaire d'Ovaire de Hamster Chinois (CHO).

L'un des systèmes de valeur est le système baculovirus, dans lequel des cellules de papillon sont co-transfectées par un vecteur d'ADN codant pour la stromélysine-3, ou un peptide approprié, et l'ADN de baculovirus. La recombinaison se produit à l'intérieur de la cellule, et les recombinants de baculovirus appropriés peuvent être choisis par des techniques standards. Par la suite, le recombinant peut être utilisé pour infecter la lignée cellulaire comme désiré, la stromélysine-3 ou le peptide étant exprimé du fait de cette infection. Un avantage particulier de ce système est la quantité de protéine produite, qui peut se situer dans la plage d'environ 1 à environ 500 mg/litre.

Bien que de tels systèmes aient tendance à ne pas être aussi aisés à utiliser que le système d'E. coli, l'avantage réside dans le traitement de la protéine après la synthèse primaire. E. coli, par exemple, n'emploie pas le même système pour le traitement des pré-proprotéines que les cellules de mammifère.

D'autres systèmes d'expression qui peuvent être employés comprennent les streptomycètes, par exemple, et les levures, telles que Saccharomyces spp., en particulier, S. cerevisiae. N'importe quel système peut être utilisé comme on le souhaite, généralement en fonction de ce qui est requis par l'opérateur. Des systèmes appropriés peuvent être également utilisés pour amplifier le matériel génétique, mais il est généralement commode d'utiliser E. coli pour cet objectif dans lequel seule la prolifération de l'ADN est requise.

Il peut être avantageux de produire uniquement l'enzyme mature, pour les objectifs de diriger des anticorps, étant donné que la séquence de l'enzyme mature est commune également aux pro- et prépro-séquences. Cependant, on comprendra que le clivage des parties pro et prépro puisse modifier la configuration tertiaire de la molécule, et il est ainsi possible qu'un anticorps dirigé contre l'enzyme mature ne détectera pas la proenzyme, par exemple. Des anticorps dirigés contre l'enzyme dans l'un ou l'autre de ses stades précoces et/ou contre les pré- ou pro-peptides qui sont clivés, peuvent également se révéler utiles.

La séquence peptidique ou nucléotidique peut être n'importe quelle séquence qui est caractéristique de stromélysine-3, en prenant en considération l'objectif pour lequel elle doit être exprimée. D'une manière idéale, les séquences devraient être complètement caractéristiques de la stromélysine-3, mais la longueur de telles séquences peut varier en fonction de la région de la molécule de stromélysine-3. Les régions que l'on préfère le plus sont celles qui sont fortement conservées, et qui ne sont pas communes avec d'autres protéines, bien qu'il puisse être avantageux que la séquence soit caractéristique des MMPs, ou, plus particulièrement, des MMPs qui sont associées à des tumeurs invasives.

L'invention comprend et porte sur des équivalents des séquences peptidiques et nucléotidiques ci-dessus, le terme "équivalent" étant utilisé au sens de la description précédente, autrement dit, des équivalents au sens de séquences ayant des substitutions au niveau des extrémités Cou N-terminales, ou n'importe où ailleurs.

L'invention comprend également des mutants des séquences, le terme "mutants" étant utilisé en rapport avec des délétions, des additions, des insertions, des inversions et des substitutions de restes d'acides aminés ou de bases dans la séquence soumise aux restrictions décrites ci-dessus.

Nous avons également découvert que l'expression de la stromélysine-3 peut être stimulée, par exemple, par des facteurs de croissance et des promoteurs de tumeurs. Des exemples typiques de tels facteurs comprennent EGF, FGF et PDGF, et TPA. Ainsi, en liaison avec les procédés précédents, la détection de l'un quelconque de ces facteurs dans un échantillon de tumeur peut également aider à diagnostiquer l'état métastatique d'un cancer.

Ainsi, l'invention propose également le traitement d'un cancer métastatique par modification de l'expression du gène de la stromélysine-3. Ceci peut être effectué par interférence avec le facteur requis pour stimuler la production de stromélysine-3, par exemple en dirigeant des anticorps spécifiques contre le facteur, lesquels anticorps peuvent encore être modifiés pour obtenir le résultat désiré. Il peut également être possible de bloquer le récepteur pour le facteur, ce qui peut être plus aisément obtenu par localisation de l'agent de liaison nécessaire, lequel peut être un anticorps ou un peptide de synthèse, par exemple.

La modification de l'expression du gène de la stromélysine-3 peut être également obtenue plus directement, telle que par blocage d'un site, tel que le promoteur, sur l'ADN génomique.

Lorsque la présente invention propose l'administration, par exemple, d'anticorps à un patient, ceci peut alors être fait par n'importe quelle voie appropriée. Si la tumeur est toujours supposée être, ou diagnostiquée comme étant, localisée, une méthode appropriée d'administration peut alors être l'injection directe dans le site. Si la cible est le cancer du sein, une injection dans le sein peut alors suffire, ou un implant peut être utilisé. Si des TIMPs doivent être administrés, par exemple, il peut être alors également possible d'employer un patch-test dermique pour une administration prolongée.

Si le cancer est un cancer du pharynx, une autre possibilité peut alors être une administration orale, par exemple, à l'aide de gargarismes.

Dans l'un ou l'autre cas, l'administration peut se faire à la place, ou additionnellement, par injection, incluant des injections sous-cutanées, intramusculaires, intraveineuses et intradermiques.

Les formulations peuvent être n'importe quelles formulations appropriées pour la voie d'administration, et apparaîtront à l'évidence à l'homme du métier. Les formulations peuvent contenir un support approprié, tel que du sérum physiologique, et peuvent également comprendre des agents donnant du volume, d'autres préparations médicinales, des adjuvants et tous autres ingrédients pharmaceutiques appropriés.

Des préparations appropriées peuvent également inclure des vaccins comprenant de la stromélysine-3 ou un peptide caractéristique de celle-ci. De tels vaccins peuvent être actifs ou passifs, mais les vaccins passifs sont généralement préférés étant donné que l'expression de la stromélysine-3 se produit dans l'utérus, et qu'une exposition indéfinie aux anticorps de stromélysine-3 peut avoir des effets indésirables. Cependant, une vaccination active peut être avantageuse, en particulier lorsqu'un patient a eu une hystérectomie, étant donné qu'aucun tissu n'exprimera alors normalement la stromélysine-3. D'autres vaccins appropriés comprennent des virus recombinants contenant une séquence nucléotidique codant pour une stromélysine-3 ou un peptide caractéristique de celle-ci. Un virus approprié de ce type est le virus de la vaccine.

Les Exemples suivants servent à illustrer la présente invention, mais ne sont pas destinés à limiter celle-ci d'une quelconque manière.

### EXEMPLE 1

### Clonage d'un ADNc spécifique du cancer du sein

Une banque d'ADNc de cancer du sein a été élaborée dans le vecteur λgt10 en utilisant de l'ARN poly(A⁺) provenant d'un échantillon de résection chirurgicale (désigné comme étant la tumeur C1) d'un cancer du sein primaire. 50 000 plaques ont été criblées d'une manière différentielle à l'aide de sondes (+) et (-) correspondant aux ADNc ayant subi une transcription inverse respectivement à partir de l'ARN poly(A⁺) de C1 et de l'ARN poly(A⁺) provenant d'un fibroadénome de sein (désigné comme étant F1).

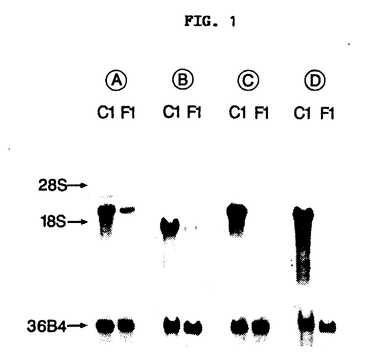

La Figure 1 représente une analyse par Northern blot d'ARN total d'un carcinome de sein C1 et d'un fibroadénome F1 à l'aide de sondes d'ADNc de quatre gènes (A-D) manifestant des taux d'expression plus élevés dans le carcinome que dans le fibroadénome. Chaque voie contenait 8 µg d'ARN total. Les filtres ont été resondés à l'aide d'une sonde 36B4 qui correspond à un gène exprimé de façon omniprésente (Rio et al., Proc. Nat. Acad. Sci. USA 84:9243-9247 (1987)).

De façon spécifique, l'ARN total a été préparé (Chirgwin et al., Biochemistry 18:5294-5299 (1979)) à partir de spécimens chirurgicaux stockés dans de l'azote liquide, et l'ARN poly(A⁺) a été choisi par chromatographie sur oligo(dT)-cellulose. Une banque d'ADNc enrichis en cancer du sein a été élaborée à l'aide d'ADNc préparé à partir d'un carcinome ductal de grade II, négatif aux récepteurs d'oestrogènes (désigné comme étant C1), dans lequel les cellules du stroma représentaient approximativement 50% de la population cellulaire totale.

Avant le clonage, l'ADNc simple brin a été soustrait avec un excès d'ARN de poly(A⁺) provenant d'un fibroadénome de sein (désigné comme étant F1), et le matériel enrichi simple brin a été purifié par chromatographie sur hydroxyapatite (Davis et al., Proc. Nat. Acad. Sci. USA 81:2194-2198 (1984) ; Rhyner et al., Neuroscience Res. 16:167-181 (1986)).

L'ADNc enrichi en cancer du sein a été rendu double brin et cloné dans le site EcoRI du vecteur λgt10. Trois millions de phages recombinants ont été obtenus, et ∼ 50 000 ont été criblés d'une manière différentielle à l'aide de filtres de Nylon réplica (Biodyne A, Pall Corporation) à partir de plaques contenant ∼ 5 000 clones d'ADNc.

Des sondes (+) et (-) ont été obtenues respectivement à l'aide d'ADNc de cancer du sein C1 et d'ADNc de fibroadénome de sein F1. Les deux sondes ont été soustraites (Davis et al., Proc. Nat. Acad. Sci. USA 81:2194-2198 (1984); Rhyner et al., Neuroscience Res. 16:167-181 (1986)) avec un excès d'ARN de foie humain total avant marquage au [³²P] en utilisant une synthèse d'amorce au hasard.

Les hybridations se sont déroulées pendant deux jours dans des conditions rigoureuses (formamide à 50%, 42°C) et un lavage a été effectué dans 2 x SSC, SDS à 0,1%, à 22°C, suivi par 0,1 x SSC, SDS à 0,1%, à 55°C. 130 plaques marquées de façon différentielle ont été choisies pour un deuxième criblage.

Les segments d'insertion d'ADNc de cinq plaques différentielles prises au hasard ont été purifiés par amplification PCR, marqués au [³²P], et hybridés avec toutes les plaques différentielles pour identifier les clones apparentés. Ce mode opératoire a été répété plusieurs fois avec des plaques différentielles prises au hasard, conduisant finalement à quatre gènes désignés comme étant A à D, qui présentaient des taux d'expression plus élevés dans le carcinome C1 que dans le fibroadénome F1. Les analyses Northern blot pour le cancer du sein C1 et le fibroadénome du sein F1 ont été préparées à l'aide de l'ARN total (8 µg) séparé par l'électrophorèse dans des gels d'agarose à 1% contenant du formaldéhyde et transféré sur des filtres Hybond-N (Amersham).

Les blots ont été colorés au bleu de méthylène avant préhybridation pour vérifier l'intégrité et les quantités d'ARN transféré. L'hybridation (18 h) et le lavage ont été effectués dans des conditions standards, comme décrit ci-dessus, à l'aide de fragments d'insertion d'ADNc marqués au [³²P] correspondants aux gènes A-D.

Les gènes A et B, qui ont été également exprimés dans un côlon normal (non représenté), n'ont pas été examinés davantage.

Bien qu'exprimé dans le côlon (non représenté), le gène C a été partiellement caractérisé en raison de son niveau élevé d'expression différentielle (Figure 1). Il a été également exprimé dans une diversité de lignées cellulaires épithéliales transformées et dans la peau humaine normale (non représenté). Le séquençage de l'ADNc de l'un des clones de C a indiqué que le gène correspondant appartient à la superfamille du gène de la kératine (données non représentées).

Enfin, le gène D (également désigné ici comme étant le gène de la stromélysine-3) a été étudié davantage, en raison de son expression différentielle marquée entre le carcinome C1 et le fibroadénome F1 (Figure 1), et également parce qu'il n'a pas été exprimé dans le côlon humain normal et dans un certain nombre d'autres tissus humains (infra).

### EXEMPLE 2

### Séquence du gène de la stromélysine-3 et de la protéine codée

Plusieurs clones indépendants ont été isolés à partir d'une banque d'ADNc λgt10 de cancer du sein C1 non-soustraite, à l'aide d'un fragment d'insertion d'ADNc de D comme sonde, et séquencés. La Figure 2 représente la séquence nucléotidique de la totalité de la longueur de l'ADNc de D et la séquence de la protéine correspondante.

Le cadre de lecture ouvert d'ADNc, codant pour une protéine d'une longueur de 488 acides aminés, est suivi par une région non traduite-3' de 714 bases contenant un signal de poly(A) addition, situé 14 bases en amont de l'extrémité 3' de l'ARN. Une méthionine d'initiation présumée est située au niveau de la position nucléotidique 10-12. Bien que l'AUG correspondant ne soit pas associé avec et situé dans une séquence qui se conforme au motif consensus de Kozak, la traduction est probablement amorcée au niveau de cet AUG, étant donné que la séquence immédiatement en aval correspond à celle pour un peptide leader hydrophobe, une caractéristique attendue (infra).

Sur la Figure 2, laquelle représente la séquence nucléotidique de l'ADNc de la stromélysine-3 et la séquence d'acides aminés déduite, les restes nucléotidiques sont numérotés dans la direction 5' à 3' et les acides aminés déduits dans le cadre de lecture ouvert sont désignés par leurs codes à une seule lettre. En partant de l'extrémité 5', les séquences nucléotidiques soulignées correspondent : au peptide signal supposé (deux sites de clivage potentiels sont marqués par des flèches) ; à la séquence PRCGVPD, caractéristique des prométalloprotéinases ; aux restes histidine conservés du domaine de liaison au zinc (Matrisian, L.M. Trends Genet. 6:21-125 (1990)) ; et à la séquence signal de poly(A) addition.

De façon spécifique, un segment d'insertion d'ADNc correspondant à la partie 3' de l'ADNc de D [250 paires de bases comprenant une région poly(AT) de 19 paires de base] a été marqué au [³²P] par une synthèse d'amorçage au hasard et utilisé pour cribler une banque d'ADNc λgt10 non-soustraite, produit par l'ARN poly(A⁺) de la tumeur du sein C1, par la méthode de Gubler et Hoffmann (Gene 25:262-269 (1983)). Plusieurs clones indépendants ont été identifiés et sous-clonés dans un vecteur de séquençage M13. La séquence d'ADN a été déterminée par la méthode didésoxy utilisant la séquénase et le coffret de réactif déaza-dGTP d'US Biochemical. La séquence a été analysée à l'aide du programme-produit logiciel PC/GENE.

### EXEMPLE 3

### Stromélysine-3, une métalloprotéinase supposée

La Figure 3 montre une comparaison des séquences d'acides aminés prédites de stromélysines humaines et de la collagénase humaine de type I.
(a) Des séquences d'acides aminés ont été alignées à l'aide d'un programme de multialignement (Higgins et al., Gene 73:237-244 (1988)). Les restes d'acides aminés identiques dans la totalité des quatre séquences sont marqués par des étoiles. Les flèches désignent des sites de clivage du peptide signal supposés de la stromélysine-3. La pointe de flèches est dirigée sur le clivage qui se produit lors de l'activation de la procollagénase de type I et des prostromélysines. Les 10 restes d'acides aminés spécifiques de la stromélysine-3 au niveau de ce site de clivage sont entourés. La séquence PRCGVPD et les restes conservés du domaine de liaison au zinc supposé sont soulignés.
(b) Gauche, régions d'analogie (en pourcentage d'identité d'acides aminés) entre la stromélysine-3, la stromélysine-1 (ST1, Whitham et al., Biochem. J. 240:913-916 (1986)), la stromélysine-2 (ST2, Muller et al., Biochem. J. 253:187-192 (1988)) et la collagénase de type I (COI, Whitham et al., Biochem. J. 240:913-916 (1986)) ;
(c) Droite, régions d'analogie entre ST1, ST2 et COI ; P indique le peptide signal et le pro-domaine ; ENZ indique le domaine correspondant aux enzymes actives matures.

Ainsi, la comparaison de la séquence protéinique dérivée avec la banque de données de Swissprot (libération 14) a montré que la nouvelle protéine appartient à la famille des métalloprotéinases de matrice sécrétées (MMPs) (Figure 3a). En conséquence, la nouvelle protéine possède un candidat de séquence leader N-terminal hydrophobe (souligné sur la Figure 2), et présente la séquence très conservée PRCGVPD (restes d'acides aminés 78-84), qui est caractéristique du prodomaine des MMPs, et de même possède le site de liaison au zinc des MMPs (restes d'acides aminés 212-225 - Fig. 3a) (Matrisian, L.M. Trends Genet. 6:121-125 (1990)).

Par analogie avec les autres membres de la famille, l'acide aminé N-terminal de la protéine mature est susceptible de correspondre à la phénylalanine 98 de la pré-proprotéine (Whitham et al., Biochem. J. 240:913-916 (1986)) (Fig. 3a). Après les alignements optimaux, l'analogie entre la protéine mature supposée est de 40% avec la stromélysine-1 (Whitham et al., Biochem. J. 240:913-916 (1986)), 38% avec la stromélysine-2 (Muller et al., Biochem. J. 253:187-192 (1988)) et de 36% avec la collagénase de type I (Goldberg et al., J. Biol. Chem. 261:6600-6605 (1986)) (Fig. 3b).

La spécificité de substrat de la nouvelle protéine n'est pas connue. Ici, elle est désignée comme étant la stromélysine-3, bien que son analogie avec la stromélysine-1 (40%) soit nettement très inférieure à celle existant entre la stromélysine-1 et la stromélysine-2 (79%), et même inférieure à l'analogie existant entre la collagénase de type I et la stromélysine-1 (53%) (Fig. 3b). Ainsi, bien que la protéine est une MMP, l'appellation "stromélysine" n'est pas nécessairement strictement précise, mais elle est commode.

En outre, en amont de la séquence PRCGVPD, il n'y a pas d'analogie significative entre la stromélysine-3 et les autres MMPs, avec lesquelles elle a été comparée (Fig. 3). Cependant, la stromélysine-3 a une séquence courte unique (restes d'acides aminés 88-97) au niveau d'une position correspondant pratiquement précisément avec le site de clivage de la proprotéine de la collagénase de type I et des stromélysines (Whitham et al., supra). De plus, la stromélysine-3, comme c'est le cas avec la collagénase de type I et les autres stromélysines, ne manifeste pas le domaine analogue à la fibronectine caractéristique des collagénases de type IV (Wilhelm et al., J. Biol. Chem. 264:17213-17221 (1989)).

### EXEMPLE 4

### Surexpression dans les carcinomes du sein

L'apparition de produits de transcription de l'ARN de stromélysine-3 a été étudiée dans des échantillons résectionnés de 30 carcinomes du sein et cinq fibroadénomes du sein.

La Figure 4 montre les analyses Northern blot d'ARNs de métalloprotéinases humaines dans les tumeurs du sein :
(a) ARN de la stromélysine-3 ;
(b) ARN de la collagénase type I (COI) ;
(c) ARN de la collagénase de type IV de 92 kD (COIV 92K) ;
(d) ARN de la collagénase de type IV de 72 kD (COIV 72K) ;
(e) ARNs de la stromélysine-1 et de la stromélysine-2 (ST1/2); et
(f) ARN de pump-1 (PUI).

L'ARN total a été préparé à partir de quatre carcinomes du sein négatifs aux récepteurs d'oestrogènes (C1, grade II ; C2, C3 et C4, grade III), six carcinomes du sein positifs aux récepteurs d'oestrogènes (C5, C8 et C9, grade II ; C6 et C7, grade III ; COI, grade I) et quatre fibroadénomes du sein (F2-F5). Chaque voie contenait 8 µg d'ARN. Le signal 36B4 correspond à l'ARN d'un gène de régulation (Fig. 1).

De façon spécifique, plusieurs analyses Northern blot ont été préparées en parallèle avec des échantillons d'ARN identiques, comme pour la Figure 1, et hybridées avec l'une ou l'autre des sondes d'ADNc suivantes : (a) segment d'insertion de 1,6 kb couvrant la partie 3' de l'ADNc de la stromélysine-3, (b) ADNc de COI, (e) ADNc de ST2 (qui subit une hybridation croisée avec l'ARN de ST1), (f) ADNc de PUI (sondes de COI, de ST2 et de PU1, gracieusement fournies par R. Breathnach, Muller et al., Biochem. J. 253:187-192 (1988)), ou les sondes oligonucléotidiques anti-sens 80mer correspondant à (c) COIV 92K (nucléotides 2144-2223, Wilhelm et al., J. Biol. Chem. 264:17213-17221 (1989)) et (d) COIV 72K (nucléotides 1937-2016, Collier et al., J. Biol. Chem. 263:6579-6587 (1988)).

Les sondes d'ADNc ont été marquées au (³²P) à l'aide d'une synthèse d'amorçage au hasard (∼5 x 10⁵ cpm/µg) et les oligonucléotides ont été marqués à l'aide d'une kination à l'extrémité 5' = 10⁸ cpm/µg). Des hybridations ont été effectuées dans des conditions rigoureuses (42°C, formamide à 50%) avec - 10' cpm/ml. Les filtres ont ensuite été lavés dans 2 x SSC, SDS à 0,1%, à 22°C, en faisant suivre par 0,1 x SSC, SDS à 0,1%, à 55°C. Une autoradiographie a été effectuée pour (a), 18 heures, (b), 20 heures, (c), (d) et (e), 4 jours, (f), 2 jours, à -80°C, avec un écran d'intensification.

L'ARNm de la stromélysine-3 a été trouvé dans la totalité des carcinomes du sein, indépendamment du fait qu'ils étaient positifs (C5-C10) ou négatifs (C1-C4) aux récepteurs d'oestradiols (RO) (Figure 4a), mais non dans les échantillons de fibroadénomes, avec une exception (F2) où le taux d'expression était analogue au taux le plus bas observé dans les carcinomes du sein.

La présence de produits de transcription d'ARN des autres membres de la famille des gènes des MMPs a également été recherchée dans les mêmes échantillons (Fig. 4b-f). Ces autres membres de la famille des MMP peuvent clairement être séparés en deux classes, en fonction de leurs motifs d'expression dans les tumeurs humaines du sein. La première classe comprend la collagénase de type IV de 72 kD (COIV 72K, Fig. 4d), la stromélysine-1 et -2 (ST1/2, Fig. 4e) et pump-1 (PU1, Fig. 4f), la totalité de ces gènes étant exprimée à la fois dans les tumeurs malignes et bénignes. Au contraire, la seconde classe, qui comprend les gènes de la stromélysine-3 (Fig. 4a), de la collagénase de type I (COI, Fig. 4b) et de la collagénase de type IV de 92 kD (COIV 92K, Fig. 4c), manifeste une surexpression seulement dans les carcinomes du sein.

Les motifs expression n'étaient pas identiques pour les trois gènes de la seconde classe. Des produits de transcription de l'ARN de la collagénase de type I n'ont pas été détectés dans les carcinomes C5, C6, C7 et CI0, et des produits de transcription de l'ARN de la collagénase de type IV de 92 kD n'ont pas été observés dans les échantillons C7 et CI0, mais des produits de transcription de l'ARN de la stromélysine-3 ont été nettement détectés dans toutes les tumeurs.

Ainsi, la stromélysine-3 apparaît être un produit de diagnostic de carcinomes du sein invasifs, alors que la collagénase de type I et la collagénase de type IV de 92 kD peuvent également être mises en jeu de façon spécifique dans l'évolution du cancer du sein dans certains cas.

### EXEMPLE 5

### Expression dans des cellules et tissus provenant de diverses sources

La Figure 5 montre des analyses par Northern blot de l'ARN de stromélysine-3 dans diverses lignées cellulaires et tissus.
(a) Trois noeuds lymphatiques auxiliaires normaux et cinq métastatiques provenant de patientes atteintes du cancer du sein ;
(b) quatre lignées de cellules de cancer du sein négatives aux récepteurs d'oestrogènes (BT-20, MDA-231, SK-BR-3, HBL-100) et quatre positives aux récepteurs d'oestrogènes (T-47D, BT-474, ZR-75-1, MCF-7) ;
(c) 10 tissus humains normaux ;
(d) fibroblastes diploïdes de foetus humain HFL-1 (ATCC CCL 153), cultivés dans un milieu exempt de sérum (1 et 2), en l'absence (1) ou en présence (2) de TPA (10 ng/ml) ou cultivés dans un milieu exempt de sérum, supplémenté par 20 µg/ml d'insuline (3 à 6), en l'absence (3) ou en présence (4) de PDGF (20 ng/ml, British Biotechnology), (5) d'EGF (20 ng/ml, Collaborative Research) ou (6) de bFGF (10 ng/ml, gracieusement fourni par Pettmann (FEBS Lett. 189:102-108 (1985)).

Dans (a), chaque voie contenait 10 µg d'ARN total à l'exception de la voie 5 (2 µg) et de la voie 6 (20 µg). Dans (b) et (c), chaque voie contenait 8 µg d'ARN total, et dans (d), chaque voie contenait 5 µg d'ARN cytoplasmique.

De façon spécifique, dans (a), (b) et (c), les blots ont été réalisés et traités comme indiqué sur la Fig. 4 pour la stromélysine-3. Dans (d), des fibroblastes HFL-1 confluents ont été conservés dans du milieu de culture DMEM exempt de sérum. Au bout de 24 heures, du milieu frais a été ajouté et supplémenté ou non par TPA ou des facteurs de croissance, comme indiqué ci-dessus. Au bout de 24 heures de culture, les cellules ont été récoltées et l'ARN cytoplasmique, préparé (Gough, N.M., Analyt. Biochem. 173:93-95 (1988)).

Les blots ont ensuite été préparés et traités comme indiqué sur la Fig. 4 pour la stromélysine-3, mais l'autoradiographie a été effectuée pendant trois jours.

Des produits de transcription de l'ARN de la collagénase IV de 92 kD ont été trouvés dans 3 noeuds lymphatiques normaux et 5 noeuds lymphatiques métastatiques de cancer du sein, alors que des produits de transcription de l'ARN de la stromélysine-3 ont été détectés seulement dans les noeuds métastatiques (Fig. 5a, et données non présentées).

Contrairement aux résultats obtenus avec des tumeurs du sein malignes primaires et des noeuds lymphatiques métastatiques, il n'a pas pu être détecté de produits de transcription de l'ARN de la stromélysine-3 dans des conditions analogues dans huit lignées de cellulaires humaines de cancer du sein, indépendamment de leur comportement positif ou négatif aux RO (Fig. 5b). De façon analogue, des produits de transcription de l'ARN de la stromélysine-3 n'ont pas pu être détectés dans un certain nombre de tissus adultes humains normaux (Fig. 5c), avec deux exceptions notables, l'utérus et le placenta.

La stromélysine-3 n'est apparemment pas associée à tous les cancers, et seuls de faibles taux de produits de transcription d'ARN de stromélysine-3 ont été trouvés dans des échantillons d'ARN provenant de cancer du côlon, de l'ovaire, du rein et du poumon. Cependant, des taux élevés d'expression, comparables à ceux trouvés dans les cancers du sein, ont été observés dans les échantillons d'ARN du cancer du larynx (données non présentées).

### EXEMPLE 6

### Expression spécifique dans des cellules du stroma de tumeurs invasives

L'expression du gène de la stromélysine-3 dans des carcinomes du sein primitifs, mais non dans un certain nombre de lignées cellulaires du cancer du sein établi, a suggéré que le gène était exprimé dans les cellules du stroma entourant la tumeur, plutôt que dans les cellules néoplasiques elles-mêmes.

Des expériences d'hybridation in situ à l'aide d'une ribosonde anti-sens de stromélysine-3 marquée au [³⁵S] ont été effectuées à l'aide de sections provenant de six carcinomes (tumeurs C1, C3, C5, C9, C10, désignées comme pour la Fig. 4, et tumeur C11, un carcinome positif aux RO non représentée sur Fig. 4).

De façon spécifique, une hybridation in situ a été effectuée comme décrit par Cox et al. (Dev. Biol. 101:485-502 (1984)). Des sections déparaffinisées et traitées par un acide (épaisseur de 6 µm) ont été traitées à la protéinase K et hybridées pendant une nuit avec des produits de transcription anti-sens marqués au [³⁵S] provenant d'un segment d'insertion d'ADNc de la stromélysine-3 (467 paires de bases s'étendant des nucléotides 1128 à 1594) sous-cloné dans Bluescript II (Stratagène). L'hybridation a été suivie par un traitement par la RNase (20 µg/ml, 30 min., 37°C) et un lavage dans des conditions rigoureuses (2 x SSC, formamide à 50%, 60°C, 2 heures), avant une autoradiographie à l'aide d'une émulsion de NTB2 (Kodak). L'autoradiographie a duré 15 jours. Il n'a pas été observé de marquage significatif au-dessus du bruit de fond dans des conditions analogues utilisant une ribosonde sens (non représentée).

La Figure 6 montre la présence de produits de transcription de l'ARN de la stromélysine-3 dans des sections de carcinome du sein et des bourgeons de membres embryonnaires. a, c, e, g, i, et k : fonds clairs de sections de tissus (x100) colorées à l'hématoxyline ; b, d, f, h, j et l : les mêmes sections (encore colorées à l'hématoxyline) après hybridation in situ avec une sonde d'ARNc de la stromélysine-3 anti-sens et imagerie à fond noir.

a et b, carcinome du sein ductal de grade II (tumeur C1, voir Fig. 4) : des cellules de cancer infiltrantes (C) sont entourées par un stroma riche en cellules fusiformes (S) ; les produits de transcription de l'ARN de la stromélysine-3 sont les plus abondants dans les cellules de stroma entourant immédiatement les cellules épithéliales néoplasiques. c et d, carcinome du sein ductal de grade III (tumeur C3, voir Fig. 4) : de multiples îlots de cellules du cancer du sein infiltrantes (C) sont entourés par des cellules de stroma ; l'expression du gène de la stromélysine-3 est plus faible dans la partie centrale de la plupart des trabécules du stroma (S), c'est-à-dire dans la région qui est la plus éloignée des cellules néoplasiques. e et f : carcinome ductal (tumeur C3, voir Fig. 4) conjointement avec deux lobules normaux (N) ; les produits de transcription de l'ARN de la stromélysine-3 ont été détectés exclusivement dans le stroma voisin des cellules de cancer infiltrantes (C), à l'exception d'une petite zone riche en lymphocytes (flèche). g et h, carcinome ductal (tumeur C10, voir Fig. 4) : les produits de transcription de l'ARN de la stromélysine-3 peuvent être détectés au-dessus du bruit de fond dans les cellules du stroma entourant les cellules du cancer du sein infiltrantes (angle supérieur droit) mais non les cellules du cancer du sein in situ (étoile). i et j, carcinome ductal (tumeur C11, positive aux RO, degré II, carcinome) ; gauche : carcinome in situ (étoiles), il n'a pas pu être détecté de produits de transcription de l'ARN de la stromélysine-3 dans les cellules de stroma ; droite : cellules néoplasiques infiltrantes entourées par les cellules de stroma exprimant le gène de la stromélysine-3. k et 1, région interdigitale d'un bourgeon de membre d'embryon humain âgé de 8 semaines : les produits de transcription de l'ARN de la stromélysine-3 sont détectés dans le mésoderme sous-jacent à l'épiderme primitif, de la façon la plus remarquable dans la zone interdigitale (M) ; notons que l'épiderme primitif (flèche), le cartilage en formation (PC), et le mésoderme environnant ne sont pas marqués.

Dans tous les cas, les produits de transcription de l'ARN de la stromélysine-3 n'ont été détectés que dans les cellules du stroma entourant les îlots de cellules épithéliales malignes qui formaient le composant invasif des tumeurs (Fig. 6 : tableaux a et b, pour la tumeur C1 ; tableaux c, d, e et f pour la tumeur C3 ; tableaux g et h pour la tumeur C10 ; tableaux i et j, côté droit, pour la tumeur C11 ; et données non présentées pour les tumeurs C5 et C9).

Dans les noeuds lymphatiques métastatiques (même source que C5), l'expression du gène de la stromélysine-3 a également été restreinte aux cellules du stroma entourant les cellules épithéliales métastatiques (données non présentées).

Il est particulièrement remarquable que, dans tous les cas, les cellules épithéliales malignes elles-mêmes n'ont pas été marquées, et que les niveaux les plus élevés d'expression ont été observés dans les cellules du stroma en apposition aux cellules malignes. En nette opposition, il n'a pas pu être détecté d'expression significative dans les cellules du stroma entourant des lésions de carcinome in situ entourées encore par une membrane basale (tableaux g et h pour la tumeur C10 ; tableaux i et j, côté gauche, pour la tumeur C11), alors que le marquage des cellules du stroma a pu être clairement observé dans le composant invasif des mêmes tumeurs (tableaux g et h pour la tumeur C10 ; tableaux i et j, côté droit, pour la tumeur C11).

De même, il n'a pas pu être détecté d'expression significative dans les cellules du stroma situées à une distance des cellules cancéreuses, ni dans les cellules du stroma entourant les vaisseaux et les canalicules normaux (par exemple, tableaux e et f). Il n'a pas été détecté de foyers discrets de produits de transcription de la stromélysine-3 dans des sections de fibroadénome F2 faiblement positives pour l'ARN de la stromélysine-3 dans les analyses par Northern blot (Fig. 4a et données non présentées).

A la fois les fibroblastes et les myofibroblastes sont connus pour être présents dans le stroma des carcinomes du sein invasifs. (Ahmed, A., Pathol. Annu. 25(Pt2):237-286 (1990)). En utilisant notre technique d'hybridation in situ, il n'a pas été possible de déterminer si seul un ou deux de ces types de cellules exprimait le gène de la stromélysine-3.

### EXEMPLE 7

### Stimulation par les facteurs de croissance

Les résultats ci-dessus indiquent que l'expression du gène de la stromélysine-3 dans les cellules du stroma est susceptible d'être induite par un facteur diffusible, sécrété par les cellules néoplasiques. Les facteurs de croissance, tels que EGF, FGF et PDGF, de même que certaines cytokines (IL-1α,β, et TNF-α), et des promoteurs de tumeurs (par exemple, TPA) sont connus par activer la transcription des gènes des MMPs (Kerr et al., Science 242:1424-1427 (1988)). Il a également été rapporté que les cellules tumorales provenant de plusieurs sources produisent un (ou des) facteur(s) qui stimule(nt) la production de collagénase I par les fibroblastes humains (Lippman et al., Recent Prog. Hormone Res. 45:383-440 (1990)). Les activités PDGF, FGF et TGF-α sont connues pour être sécrétées par les cellules de cancer du sein in vitro (Salomon et al., dans Breast cancer ; cellular and molecular biology (éds., Lippmann, M.E. et Dickson, R.B.), pages 363-389 (Kluwer, Boston, (1988)).

Pour rechercher si l'expression du gène de la stromélysine-3 pouvait être modulée par des stimuli exogènes, des fibroblastes diploïdes de foetus humains ont été développés en l'absence ou en présence de l'un ou l'autre parmi PDGF, bFGF et EGF, et également du promoteur de tumeur TPA. L'addition de l'un ou l'autre de ces facteurs de croissance ou du TPA a conduit à une augmentation des produits de transcription de l'ARN de la stromélysine-3 dans les fibroblastes, la stimulation la plus forte étant observée avec bFGF (Fig. 5d).

### EXEMPLE 8

### Expression de la stromélysine-3 dans l'embryon

Comme le gène de la stromélysine-3 a été exprimé en réponse à une stimulation par les facteurs de croissance dans les fibroblastes foetaux, nous avons recherché si le gène pouvait être normalement exprimé pendant le développement de l'embryon ou non.

Des produits de transcription de la stromélysine-3 ont été détectés dans plusieurs régions discrètes d'un embryon humain âgé de 8 semaines, de façon notable dans la région interdigitale des bourgeons de membres (Fig. 6, tableaux k et 1, et données non présentées), une région connue pour être associée à une mort cellulaire programmée à ce stade de l'embryogenèse (Milaire, J. Organogenesis (éds., De Haan, R.L. et Ursprung, H.), pages 283-300 (Holt, Rinehart et Winston, New York, 1965)).

Le marquage a été observé dans le mésoderme embryonnaire sous-jacent à l'épiderme primitif qui restait non marqué. De façon notable, les cellules mésenchymateuses situées à une certaine distance de l'épiblaste ont été également négatives aux ARNm.

Ainsi, la découverte que le gène de la stromélysine-3 est exprimé pendant le développement embryonnaire normal dans une zone où le remodelage du tissu est bien illustré par des documents de la littérature, suggère que l'expression de la stromélysine-3 dans la tumeur du sein joue un rôle dans les processus de remodelage de la MEC, associés à l'évolution des cancers.

### EXEMPLE 9

### Clonage de l'ADNc de souris codant pour la ST3

Une sonde contenant de l'ADNc humain codant pour la ST3 a été utilisée pour cribler une banque d'ADNc de placenta de souris à l'aide de modes opératoires standards (Sambrook et al., Molecular cloning ; A Laboratory Manual, Cold Spring Harbor Press (1989)). Le criblage a conduit à l'obtention d'un ADNc de souris complet codant pour la ST3 (Figure 7).

Une analyse des deux séquences a révélé une homologie de 89% dans la séquence des acides aminés de la forme mature de la ST3 et approximativement une homologie de 55% au niveau des pré- et pro-domaines (Figure 7).

Le motif d'expression dans diverses cellules de souris a été déterminé à l'aide des méthodes décrites dans les Exemples 4-8.

Le motif de l'expression de la ST3 dans la souris a été trouvé être identique en ce qui concerne la spécificité des tissus à celui trouvé avec le tissu humain. Le niveau d'expression le plus élevé a été trouvé dans le tissu placentaire et le tissu utérin.

## Revendications (Revendications pour l'(les) Etat(s) contractant(s) suivant(s): AT, BE, CH, DE, DK, FR, GB, IT, LI, LU, NL, SE)

1. Procédé pour le diagnostic d'une tumeur maligne, caractérisé par le fait qu'il comprend la détection, soit de la stromélysine-3, soit d'une séquence nucléotidique codant pour la stromélysine-3, dans un échantillon biologique, in vitro.

2. Procédé selon la revendication 1, caractérisé par le fait que la tumeur maligne est choisie dans le groupe constitué par le cancer du sein ou du pharynx, et les carcinomes de la tête, du cou et de la peau.

3. Procédé selon l'une quelconque des revendications 1 ou 2, caractérisé par le fait qu'il comprend la détection de la stromélysine-3, dans lequel un anticorps, ou un équivalent d'anticorps, spécifique de la stromélysine-3, est amené en contact avec l'échantillon, ou une préparation de celui-ci, en faisant suivre par la détection de la liaison.

4. Procédé selon la revendication 3, caractérisé par le fait que l'anticorps, ou l'équivalent d'anticorps, est marqué.

5. Un anticorps, ou des équivalents d'anticorps tels que définis à l'une quelconque des revendications 3 et 4.

6. Procédé selon l'une quelconque des revendications 1 ou 2, caractérisé par le fait qu'une séquence nucléotidique est détectée au moyen d'une sonde nucléotidique, et de l'essai d'hybridation.

7. Procédé selon la revendication 6, caractérisé par le fait que la sonde, ou le brin soeur de celle-ci, code pour une partie caractéristique ou pour la totalité de la stromélysine-3.

8. Procédé selon la revendication 7, caractérisé par le fait que la sonde, ou son brin soeur, ne code ni pour le pré- ni pour le pro-peptide de la stromélysine-3.

9. Procédé selon l'une quelconque des revendications 6 à 8, caractérisé par le fait que la sonde, ou son brin soeur, présente une partie ou la totalité de la séquence d'ADNc codant pour la stromélysine-3, ou correspond à celle-ci par la dégénérescence du code génétique, ou s'hybride à celle-ci dans les conditions d'essai.

10. Procédé selon l'une quelconque des revendications 6 à 9, caractérisé par le fait que la sonde est radiomarquée.

11. Procédé selon l'une quelconque des revendications 6 à 10, caractérisé par le fait que la sonde est une ribosonde.

12. Peptide synthétique ou exprimé comprenant une séquence d'acides aminés correspondant à une partie caractéristique ou à la totalité de la séquence d'ADNc codant pour la pré-prostromélysine-3, ou à un mutant, un variant ou un fragment de celle-ci, incluant une séquence animale correspondant à ladite séquence d'acides aminés.

13. Peptide selon la revendication 12, caractérisé par le fait que ladite séquence d'acides aminés est choisie dans le groupe constitué par la pré-prostromélysine-3 de souris et et la pré-prostromélysine-3 humaine.

14. Peptide selon l'une quelconque des revendications 12 et 13, caractérisé par le fait que ledit fragment est la prostromélysine-3.

15. Peptide selon l'une quelconque des revendications 12 et 13, caractérisé par le fait que ledit fragment est la stromélysine-3 mature.

16. Séquence d'ADNc codant pour un peptide tel que défini à l'une quelconque des revendications 12 à 15.

17. Vecteur comprenant une séquence telle que définie à la revendication 16.

18. Système d'expression pour la production d'un peptide tel que défini à la revendication 12, comprenant une cellule hôte appropriée et un vecteur tel que défini à la revendication 17, dans lequel le vecteur est un vecteur d'expression.

19. Procédé de fabrication d'un peptide tel que défini à la revendication 12, caractérisé par le fait qu'il comprend l'utilisation d'un système d'expression tel que défini à la revendication 18, et l'isolement du peptide désiré.

20. Utilisation d'un agent capable d'affecter spécifiquement l'expression du gène de la stromélysine-3, ou d'affecter spécifiquement l'activité stromélysine-3, dans la fabrication d'un médicament pour le traitement ou la prophylaxie d'un cancer métastatique, en particulier le cancer du sein ou du pharynx, et les carcinomes de la tête, du cou et de la peau.

21. Utilisation selon la revendication 20, caractérisée par le fait que le cancer est le cancer du sein.

22. Utilisation selon l'une quelconque des revendications 20 et 21, caractérisée par le fait que l'agent est un anticorps, ou un équivalent d'anticorps, spécifique de la stromélysine-3.

23. Utilisation selon la revendication 22, caractérisée par le fait que l'anticorps, ou l'équivalent d'anticorps, porte un marqueur toxique.

24. Utilisation selon la revendication 23, caractérisée par le fait que le marqueur est un radioisotope.

25. Utilisation selon l'une quelconque des revendications 20 et 21, caractérisée par le fait que l'agent est un inhibiteur tissulaire ou autre, de préférence synthétique, de métalloprotéinase spécifique de la stromélysine-3.

26. Utilisation selon l'une quelconque des revendications 20 et 21, caractérisée par le fait que l'agent modifie l'activité de la stromélysine-3.

27. Utilisation selon l'une quelconque des revendications 20 et 21, caractérisée par le fait que l'agent empêche l'expression du gène de la stromélysine-3.

28. Utilisation selon l'une quelconque des revendications 20 et 21, caractérisée par le fait que l'agent empêche la maturation de la pré-proprotéine en stromélysine-3.

29. Agent tel que défini à l'une quelconque des revendications 20 à 28, pour utilisation en thérapie, en particulier en thérapie du cancer.

30. Vaccin comprenant un peptide tel que défini à la revendication 12, ou un virus contenant une séquence nucléotidique codant pour un tel peptide, conjointement avec un support approprié pour celui-ci.

## Revendications (Revendications pour l'(les) Etat(s) contractant(s) suivant(s): ES, GR)

1. Procédé pour le diagnostic d'une tumeur maligne, caractérisé par le fait qu'il comprend la détection, soit de la stromélysine-3, soit d'une séquence nucléotidique codant pour la stromélysine-3, dans un échantillon biologique, in vitro.

2. Procédé selon la revendication 1, caractérisé par le fait que la tumeur maligne est choisie dans le groupe constitué par le cancer du sein ou du pharynx, et les carcinomes de la tête, du cou et de la peau.

3. Procédé selon l'une quelconque des revendications 1 et 2, caractérisé par le fait qu'il comprend la détection de la stromélysine-3, dans lequel un anticorps, ou un équivalent d'anticorps, spécifique de la stromélysine-3, est amené en contact avec l'echantillon, ou une préparation de celui-ci, en faisant suivre par la détection de la liaison.

4. Procédé selon la revendication 3, caractérisé par le fait que l'anticorps, ou l'équivalent d'anticorps, est marqué.

5. Procedé de fabrication d'un anticorps, ou des équivalents d'anticorps tels que definis à l'une quelconque des revendications 3 et 4, caractérisé par le fait qu'un peptide de stromélysine-3 est présenté conjointement avec une protéine support à un systeme animal, ou, si le peptide est suffisament long, sans support.

6. Procédé selon l'une quelconque des revendications 1 et 2, caractérisé par le fait qu'une séquence nucléotidique est detectée au moyen d'une sonde nucléotidique, et de l'essai d'hybridation.

7. Procédé selon la revendicatoin 6, caractérisé par le fait que la sonde, our le brin soeur de celle-ci, code pour une partie caractéristique ou pour la totalité de la stromélysine-3.

8. Procédé selon la revendication 7, caractérisé par le fait que la sonde, ou son brin soeur, ne code ni pour le pré- ni pour le pro-peptide de la stromélysine-3.

9. Procédé selon l'une quelconque des revendications 6 à 8, caractérisé par le fait que la sonde, ou son brin soeur, présente une partie ou la totalité de la séquence d'ADNc codant pour la stromélysine-3, ou correspond à celle-ci par la dégénérescence du code génétique, ou s'hybride à celle-ci dans les conditions d'essai.

10. Procédé selon l'une quelconque des revendications 6 à 9, caractérisé par le fait que la sonde est radiomarquée.

11. Procédé selon l'une quelconque des revendications 6 à 10, caractérisé par le fait que la sonde est une ribosonde.

12. Procédé de fabrication d'un peptide synthétique ou exprimé comprenant une séquence d'acides aminés correspondant à une partie caractéristique ou à la totalité de la séquence d'ADNc codant pour la pré-prostromélysine-3, ou à un mutant, un variant ou un fragment de celle-ci, incluant une séquence animale correspondant à ladite séquence d'acides aminés, caractérisé par le fait qu'il comprend l'expression de ladite séquence d'ADNc.

13. Procédé selon la revendication 12, caractérisé par le fait que ladite séquence d'acides aminés est choisie dans le groupe constitué par la pré-prostromélysine-3 de souris et la pré-prostromélysine-3 humaine.

14. Procédé selon l'une quelconque des revendications 12 et 13, caractérisé par le fait que ledit fragment est la prostromélysine-3.

15. Procédé selon l'une quelconque des revendications 12 et 13, caractérisé par le fait que ledit fragment est la stromélysine-3 mature.

16. Procédé de fabrication d'une séquence d'ADNc codant pour un peptide fabriqué par le procédé selon l'une quelconque des revendications 12 a 15, le procédé comprenant l'association successive des nucléotides.

17. Procédé de fabrication d'un vecteur comprenant une séquence fabriqué par le procédé selon la revendication 16, comprenant l'insertion dans un vecteur de la séquence d'ADNc.

18. Procédé de fabrication d'un système d'expression pour la production d'un peptide tel que défini à la revendication 12, comprenant une cellule hôte appropriée et un vecteur tel que défini à la revendication 17, dans lequel le vecteur est un vecteur d'expression, comprenant le transformation de la hôte.

19. Utilisation d'un agent capable d'affecter spécifiquement l'expression du gène de la stromélysine-3, ou d'affecter spécifiquement l'activité stromélysine-3, dans la fabrication d'un médicament pour le traitement ou la prophylaxie d'un cancer métastatique, en particulier le cancer du sein ou du pharynx, et les carcinomes de la tête, du cou et de la peau.

20. Utilisation selon la revendication 19, caractérisée par le fait que le cancer est le cancer du sein.

21. Utilisation selon l'une quelconque des revendications 19 et 20, caractérisée par le fait que l'agent est un anticorps, ou un équivalent d'anticorps, spécifique de la stromélysine-3.

22. Utilisation selon la revendication 21, caractérisée par le fait que l'anticorps, ou l'équivalent d'anticorps, porte un marqueur toxique.

23. Utilisation selon la revendication 22, caractérisée par le fait que le marqueur est un radioisotope.

24. Utilisation selon l'une quelconque des revendications 19 et 20, caractérisée par le fait que l'agent est un inhibiteur tissulaire ou autre, de préférence synthétique, de métalloprotéinase spécifique de la stromélysine-3.

25. Utilisation selon l'une quelconque des revendications 19 et 20, caractérisée par le fait que l'agent modifie l'activité de la stromélysine-3.

26. Utilisation selon l'une quelconque des revendications 19 et 20, caractérisée par le fait que l'agent empêche l'expression du gène de la stromélysine-3.

27. Utilisation selon l'une quelconque des revendications 19 et 20, caractérisée par le fait que l'agent empêche la maturation de la pré-proprotéine stromélysine-3.

28. Procédé de fabrication d'un vaccin comprenant mélanger un peptide tel que défini à la revendication 12, ou un virus contenant une séquence nucléotidique codant pour un tel peptide conjointement avec un support approprié pour celui-ci.

## Patentansprüche (Patentansprüche für folgende(n) Vertragsstaat(en): AT, BE, CH, DE, DK, FR, GB, IT, LI, LU, NL, SE)

1. Verfahren zur Diagnose eines malignen Tumors, dadurch gekennzeichnet, daß es den In-vitro-Nachweis von entweder Stromelysin-3 oder einer für das Stromelysin-3 codierenden Nucleotidsequenz in einer biologischen Probe umfaßt.

2. Verfahren nach Anspruch 1, dadurch gekennzeichnet, daß der maligne Tumor aus der Gruppe bestehend aus Brust- oder Pharynxkrebs und den Karzinomen des Kopfes, des Halses und der Haut ausgewählt wird.

3. Verfahren nach einem der Ansprüche 1 oder 2, dadurch gekennzeichnet, daß es den Nachweis von Stromelysin-3 umfaßt, bei dem ein Antikörper oder ein Antikörperäquivalent, der/das für das Stromelysin-3 spezifisch ist, mit der Probe oder einer Präparation davon in Kontakt gebracht wird, gefolgt vom Nachweis der Bindung.

4. Verfahren nach Anspruch 3, dadurch gekennzeichnet, daß der Antikörper oder das Antikörperäquivalent markiert ist.

5. Antikörper oder Antikörperäquivalente, wie sie in einem der Ansprüche 3 und 4 definiert sind.

6. Verfahren nach einem der Ansprüche 1 oder 2, dadurch gekennzeichnet, daß eine Nucleotidsequenz mittels einer Nucleotidsonde und des Hybridisierungstests nachgewiesen wird.

7. Verfahren nach Anspruch 6, dadurch gekennzeichnet, daß die Sonde oder der komplementäre Strang derselben für einen charakteristischen Teil oder für die Gesamtheit vom Stromelysin-3 codiert.

8. Verfahren nach Anspruch 7, dadurch gekennzeichnet, daß die Sonde oder ihr komplementärer Strang weder für das Prä- noch für das Propeptid des Stromelysin-3 codiert.

9. Verfahren nach einem der Ansprüche 6 bis 8, dadurch gekennzeichnet, daß die Sonde oder ihr komplementärer Strang einen Teil oder die Gesamtheit der für das Stromelysin-3 codierenden cDNA-Sequenz aufweist oder dieser hinsichtlich der Degeneration des genetischen Codes entspricht oder unter den Testbedingungen an diese hybridisiert.

10. Verfahren nach einem der Ansprüche 6 bis 9, dadurch gekennzeichnet, daß die Sonde radioaktiv markiert ist.

11. Verfahren nach einem der Ansprüche 6 bis 10, dadurch gekennzeichnet, daß die Sonde eine RNA-Sonde ist.

12. Synthetisches oder exprimiertes Peptid, das eine Aminosäuresequenz umfaßt, die einem charakteristischen Teil oder der Gesamtheit der für das Präprostromelysin-3 codierenden cDNA-Sequenz oder einer Mutante, Variante oder einem Fragment davon entspricht, einschließlich einer dieser Aminosäuresequenz entsprechenden tierischen Sequenz.

13. Peptid nach Anspruch 12, dadurch gekennzeichnet, daß die Aminosäuresequenz aus der Gruppe bestehend aus dem Präprostromelysin-3 von Mäusen und dem menschlichen Präprostromelysin-3 gewählt wird.

14. Peptid nach einem der Ansprüche 12 und 13, dadurch gekennzeichnet, daß das Fragment das Prostromelysin-3 ist.

15. Peptid nach einem der Ansprüche 12 und 13, dadurch gekennzeichnet, daß das Fragment das reife Stromelysin-3 ist.

16. cDNA-Sequenz, die für ein Peptid codiert, wie es in einem der Ansprüche 12 bis 15 definiert ist.

17. Vektor, der eine Sequenz enthält, wie sie in Anspruch 16 definiert ist.

18. Expressionssystem für die Produktion eines Peptids, wie es in Anspruch 12 definiert ist, umfassend eine geeignete Wirtszelle und einen Vektor, wie er in Anspruch 17 definiert ist, wobei der Vektor ein Expressionsvektor ist.

19. Verfahren zur Herstellung eines Peptids, wie es in Anspruch 12 definiert ist, dadurch gekennzeichnet, daß es die Verwendung eines Expressionssystems, wie es in Anspruch 18 definiert ist, und die Isolation des gewünschten Peptids umfaßt.

20. Verwendung eines Wirkstoffs, der imstande ist, spezifisch auf die Expression des Stromelysin-3-Gens einzuwirken oder spezifisch auf die Aktivität vom Stromelysin-3 einzuwirken, bei der Herstellung eines Medikaments zur Vorbeugung gegen oder Behandlung eines metastatischen Krebses, insbesondere des Brust- oder Pharynxkrebses und der Karzinome des Kopfes, des Halses und der Haut.

21. Verwendung nach Anspruch 20, dadurch gekennzeichnet, daß der Krebs Brustkrebs ist.

22. Verwendung nach einem der Ansprüche 20 und 21, dadurch gekennzeichnet, daß der Wirkstoff ein Antikörper oder ein Antikörperäquivalent ist, der/das für das Stromelysin-3 spezifisch ist.

23. Verwendung nach Anspruch 22, dadurch gekennzeichnet, daß der Antikörper oder das Antikörperäquivalent einen toxischen Marker trägt.

24. Verwendung nach Anspruch 23, dadurch gekennzeichnet, daß der Marker ein Radioisotop ist.

25. Verwendung nach einem der Ansprüche 20 und 21, dadurch gekennzeichnet, daß der Wirkstoff ein geweblicher oder ein anderer, vorzugsweise synthetischer Inhibitor einer für das Stromelysin-3 spezifischen Metalloproteinase ist.

26. Verwendung nach einem der Ansprüche 20 und 21, dadurch gekennzeichnet, daß der Wirkstoff die Aktivität vom Stromelysin-3 modifiziert.

27. Verwendung nach einem der Ansprüche 20 und 21, dadurch gekennzeichnet, daß der Wirkstoff die Expression des Stromelysin-3-Gens verhindert.

28. Verwendung nach einem der Ansprüche 20 und 21, dadurch gekennzeichnet, daß der Wirkstoff die Reifung des Präproproteins zum Stromelysin-3 verhindert.

29. Wirkstoff, wie er in einem der Ansprüche 20 bis 28 definiert ist, für die Verwendung zur Therapie, insbesondere zur Krebstherapie.

30. Impfstoff, der ein Peptid, wie es in Anspruch 12 definiert ist, oder ein Virus, das eine für ein solches Peptid codierende Nucleotidsequenz enthält, gemeinsam mit einem dafür geeigneten Träger umfaßt.

## Patentansprüche (Patentansprüche für folgende(n) Vertragsstaat(en): ES, GR)

1. Verfahren zur Diagnose eines malignen Tumors, dadurch gekennzeichnet, daß es den In-vitro-Nachweis von entweder Stromelysin-3 oder einer für das Stromelysin-3 codierenden Nucleotidsequenz in einer biologischen Probe umfaßt.

2. Verfahren nach Anspruch 1, dadurch gekennzeichnet, daß der maligne Tumor aus der Gruppe bestehend aus Brust- oder Pharynxkrebs und den Karzinomen des Kopfes, des Halses und der Haut ausgewählt wird.

3. Verfahren nach einem der Ansprüche 1 und 2, dadurch gekennzeichnet, daß es den Nachweis von Stromelysin-3 umfaßt, bei dem ein Antikörper oder ein Antikörperäquivalent, der/das für das Stromelysin-3 spezifisch ist, mit der Probe oder einer Präparation davon in Kontakt gebracht wird, gefolgt vom Nachweis der Bindung.

4. Verfahren nach Anspruch 3, dadurch gekennzeichnet, daß der Antikörper oder das Antikörperäquivalent markiert ist.

5. Verfahren zur Herstellung eines Antikörpers oder der Antikörperäquivalente, wie sie in einem der Ansprüche 3 und 4 definiert sind, dadurch gekennzeichnet, daß ein Peptid von Stromelysin-3 gemeinsam mit einem Trägerprotein oder, wenn das Peptid genügend lang ist, ohne Träger einem tierischen System dargeboten wird.

6. Verfahren nach einem der Ansprüche 1 und 2, dadurch gekennzeichnet, daß eine Nucleotidsequenz mittels einer Nucleotidsonde und des Hybridisierungstests nachgewiesen wird.

7. Verfahren nach Anspruch 6, dadurch gekennzeichnet, daß die Sonde oder der komplementäre Strang derselben für einen charakteristischen Teil oder für die Gesamtheit vom Stromelysin-3 codiert.

8. Verfahren nach Anspruch 7, dadurch gekennzeichnet, daß die Sonde oder ihr komplementärer Strang weder für das Prä- noch für das Propeptid des Stromelysin-3 codiert.

9. Verfahren nach einem der Ansprüche 6 bis 8, dadurch gekennzeichnet, daß die Sonde oder ihr komplementärer Strang einen Teil oder die Gesamtheit der für das Stromelysin-3 codierenden cDNA-Sequenz aufweist oder dieser hinsichtlich der Degeneration des genetischen Codes entspricht oder unter den Testbedingungen an diese hybridisiert.

10. Verfahren nach einem der Ansprüche 6 bis 9, dadurch gekennzeichnet, daß die Sonde radioaktiv markiert ist.

11. Verfahren nach einem der Ansprüche 6 bis 10, dadurch gekennzeichnet, daß die Sonde eine RNA-Sonde ist.

12. Verfahren zur Herstellung eines synthetischen oder exprimierten Peptids, das eine Aminosäuresequenz umfaßt, die einem charakteristischen Teil oder der Gesamtheit der für das Präprostromelysin-3 codierenden cDNA-Sequenz oder einer Mutante, Variante oder einem Fragment davon entspricht, einschließlich einer dieser Aminosäuresequenz entsprechenden tierischen Sequenz, dadurch gekennzeichnet, daß es die Expression dieser cDNA-Sequenz umfaßt.

13. Verfahren nach Anspruch 12, dadurch gekennzeichnet, daß die Aminosäuresequenz aus der Gruppe bestehend aus dem Präprostromelysin-3 von Mäusen und dem menschlichen Präprostromelysin-3 gewählt wird.

14. Verfahren nach einem der Ansprüche 12 und 13, dadurch gekennzeichnet, daß das Fragment das Prostromelysin-3 ist.

15. Verfahren nach einem der Ansprüche 12 und 13, dadurch gekennzeichnet, daß das Fragment das reife Stromelysin-3 ist.

16. Verfahren zur Herstellung einer cDNA-Sequenz, die für ein mittels des Verfahrens nach einem der Ansprüche 12 bis 15 hergestelltes Peptid codiert, wobei das Verfahren die aufeinanderfolgende Verbindung von Nucleotiden umfaßt.

17. Verfahren zur Herstellung eines Vektors, der eine mittels des Verfahrens nach Anspruch 16 hergestellte Sequenz enthält, das die Insertion der cDNA-Sequenz in einen Vektor umfaßt.

18. Verfahren zur Herstellung eines Expressionssystems für die Produktion eines Peptids, wie es in Anspruch 12 definiert ist, umfassend eine geeignete Wirtszelle und einen Vektor, wie er in Anspruch 17 definiert ist, wobei der Vektor ein Expressionsvektor ist, Verfahren, das die Transformation des Wirtes umfaßt.

19. Verwendung eines Wirkstoffs, der imstande ist, spezifisch auf die Expression des Stromelysin-3-Gens einzuwirken oder spezifisch auf die Aktivität vom Stromelysin-3 einzuwirken, bei der Herstellung eines Medikaments zur Vorbeugung gegen oder Behandlung eines metastatischen Krebses, insbesondere des Brust- oder Pharynxkrebses und der Karzinome des Kopfes, des Halses und der Haut.

20. Verwendung nach Anspruch 19, dadurch gekennzeichnet, daß der Krebs Brustkrebs ist.

21. Verwendung nach einem der Ansprüche 19 und 20, dadurch gekennzeichnet, daß der Wirkstoff ein Antikörper oder ein Antikörperäquivalent ist, der/das für das Stromelysin-3 spezifisch ist.

22. Verwendung nach Anspruch 21, dadurch gekennzeichnet, daß der Antikörper oder das Antikörperäquivalent einen toxischen Marker trägt.

23. Verwendung nach Anspruch 22, dadurch gekennzeichnet, daß der Marker ein Radioisotop ist.

24. Verwendung nach einem der Ansprüche 19 und 20, dadurch gekennzeichnet, daß der Wirkstoff ein geweblicher oder ein anderer, vorzugsweise synthetischer Inhibitor einer für das Stromelysin-3 spezifischen Metalloproteinase ist.

25. Verwendung nach einem der Ansprüche 19 und 20, dadurch gekennzeichnet, daß der Wirkstoff die Aktivität vom Stromelysin-3 modifiziert.

26. Verwendung nach einem der Ansprüche 19 und 20, dadurch gekennzeichnet, daß der Wirkstoff die Expression des Stromelysin-3-Gens verhindert.

27. Verwendung nach einem der Ansprüche 19 und 20, dadurch gekennzeichnet, daß der Wirkstoff die Reifung des Präproproteins zum Stromelysin-3 verhindert.

28. Verfahren zur Herstellung eines Impfstoffs, welches das Mischen eines Peptids, wie es in Anspruch 12 definiert ist, oder eines Virus, das eine für ein solches Peptid codierende Nucleotidsequenz enthält, mit einem dafür geeigneten Träger umfaßt.

## Claims (Claims for the following Contracting State(s): AT, BE, CH, DE, DK, FR, GB, IT, LI, LU, NL, SE)

1. Method for diagnosing a malignant tumour, characterised in that it comprises the detection either of stromelysine-3 or of a nucleotide sequence coding for stromelysine-3, in a biological sample, in vitro.

2. Method according to claim 1, characterised in that the malignant tumour is selected from the group caused by cancer of the breast or pharynx and carcinomas of the head, neck and skin.

3. Method according to one of claims 1 or 2, characterised in that it comprises the detection of stromelysine-3, wherein an antibody or antibody equivalent specific to stromelysine-3 is brought into contact with the sample or a preparation thereof, followed by the detection of bonding.

4. Method according to claim 3, characterised in that the antibody or antibody equivalent is marked.

5. An antibody or antibody equivalents as defined in one of claims 3 and 4.

6. Method according to one of claims 1 or 2, characterised in that a nucleotide sequence is detected by means of a nucleotide probe and the hybridisation test.

7. Method according to claim 6, characterised in that the probe or the sister strand thereof codes for a characteristic part or for the whole of the stromelysine-3.

8. Method according to claim 7, characterised in that the probe or its sister strand codes neither for the pre- nor for the pro-peptide of stromelysine-3.

9. Method according to one of claims 6 to 8, characterised in that the probe or its sister strand has part or all of the cDNA sequence coding for stromelysine-3 or corresponds thereto by the degeneracy of the genetic code, or hybridises therewith in test conditions.

10. Method according to one of claims 6 to 9, characterised in that the probe is radiomarked.

11. Method according to one of claims 6 to 10, characterised in that the probe is a riboprobe.

12. Synthetic or expressed peptide comprising an amino acid sequence corresponding to a characteristic part or to the whole of the cDNA sequence coding for pre-prostromelysine-3 or to a mutation, variation or fragment thereof, including an animal sequence corresponding to the said amino acid sequence.

13. Peptide according to claim 12, characterised in that the said amino acid sequence is selected from the group comprised of pre-prostromelysine-3 of mice and human pre-prostromelysine-3.

14. Peptide according to one of claims 12 and 13, characterised in that the said fragment is prostromelysine-3.

15. Peptide according to one of claims 12 and 13, characterised in that the said fragment is mature stromelysine-3.

16. Sequence of cDNA coding for a peptide as defined in one of claims 12 to 15.

17. Vector comprising a sequence as defined in claim 16.

18. System of expression for the production of a peptide as defined in claim 12, comprising an appropriate host cell and a vector as defined in claim 17, wherein the vector is an expression vector.

19. Method for producing a peptide as defined in claim 12, characterised in that it comprises the use of a system of expression as defined in claim 18, and the isolation of the desired peptide.

20. Use of an agent able to accomplish specifically the expression of the stromelysine-3 gene or to accomplish specifically the stromelysine-3 activity, in the production of a medicament for the treatment or prophylaxis of a metastatic cancer, in particular cancer of the breast or pharynx, and carcinomas of the head, neck and skin.

21. Use according to claim 20, characterised in that the cancer is breast cancer.

22. Use according to one of claims 20 and 21, characterised in that the agent is an antibody or antibody equivalent specific to stromelysine-3.

23. Use according to claim 22, characterised in that the antibody or antibody equivalent has a toxic marker.

24. Use according to claim 23, characterised in that the marker is a radioisotope .

25. Use according to one of claims 20 and 21, characterised in that the agent is a tissue inhibiter or the like, preferably synthetic, of metalloproteinase specific to stromelysine-3.

26. Use according to one of claims 20 and 21, characterised in that the agent modifies the activity of stromelysine-3.

27. Use according to one of claims 20 and 21, characterised in that the agent prevents the expression of the stromelysine-3 gene.

28. Use according to one of claims 20 and 21, characterised in that the agent prevents the maturation of the pre-proprotein into stromelysine-3.

29. Agent as defined in one of claims 20 to 28, for use in therapy, in particular in cancer therapy.

30. Vaccine comprising a peptide as defined in claim 12, or a virus containing a nucleotide sequence coding for such a peptide, conjointly with an appropriate support medium therefor.

## Claims (Claims for the following Contracting State(s): ES, GR)

1. Method for diagnosing a malignant tumour, characterised in that it comprises the detection either of stromelysine-3 or of a nucleotide sequence coding for stromelysine-3, in a biological sample, in vitro.

2. Method according to claim 1, characterised in that the malignant tumour is selected from the group caused by cancer of the breast or pharynx and carcinomas of the head, neck and skin.

3. Method according to one of claims 1 and 2, characterised in that it comprises the detection of stromelysine-3, wherein an antibody or antibody equivalent specific to stromelysine-3 is brought into contact with the sample or a preparation thereof, followed by the detection of bonding.

4. Method according to claim 3, characterised in that the antibody or antibody equivalent is marked.

5. Method for producing an antibody or antibody equivalents as defined in one of claims 3 and 4, characterised in that a peptide of stromelysine-3 is introduced conjointly with a support protein medium into an animal system or, if the peptide is sufficiently long, without a support medium.

6. Method according to one of claims 1 or 2, characterised in that a nucleotide sequence is detected by means of a nucleotide probe and the hybridisation test.

7. Method according to claim 6, characterised in that the probe or the sister strand thereof codes for a characteristic part or for the whole of the stromelysine-3.

8. Method according to claim 7, characterised in that the probe or its sister strand codes neither for the pre- nor for the pro-peptide of stromelysine-3.

9. Method according to one of claims 6 to 8, characterised in that the probe or its sister strand has part or all of the cDNA sequence coding for stromelysine-3 or corresponds thereto by the degeneracy of the genetic code, or hybridises therewith in test conditions.

10. Method according to one of claims 6 to 9, characterised in that the probe is radiomarked.

11. Method according to one of claims 6 to 10, characterised in that the probe is a riboprobe.

12. Method for producing a synthetic or expressed peptide comprising an amino acid sequence corresponding to a characteristic part or to the whole of the cDNA sequence coding for pre-prostromelysine-3 or to a mutation, variation or fragment thereof, including an animal sequence corresponding to the said amino acid sequence, characterised in that it comprises the expression of the said cDNA sequence.

13. Method according to claim 12, characterised in that the said amino acid sequence is selected from the group comprised of pre-prostromelysine-3 of mice and human pre-prostromelysine-3.

14. Method according to one of claims 12 and 13, characterised in that the said fragment is prostromelysine-3.

15. Method according to one of claims 12 and 13, characterised in that the said fragment is mature stromelysine-3.

16. Method for producing a cDNA sequence coding for a peptide produced by the method according to one of claims 12 to 15, the method comprising the successive association of the nucleotides.

17. Method for producing a vector comprising a sequence produced by the method according to claim 16, comprising the insertion of the cDNA sequence in a vector.

18. Method for producing a system of expression for the production of a peptide as defined in claim 12, comprising an appropriate host cell and a vector as defined in claim 17, wherein the vector is an expression vector, comprising the transformation of the host.

19. Use of an agent able to accomplish specifically the expression of the stromelysine-3 gene or to accomplish specifically the stromelysine-3 activity, in the production of a medicament for the treatment or prophylaxis of a metastatic cancer, in particular cancer of the breast or pharynx, and carcinomas of the head, neck and skin.

20. Use according to claim 19, characterised in that the cancer is breast cancer.

21. Use according to one of claims 19 and 20, characterised in that the agent is an antibody or antibody equivalent specific to stromelysine-3.

22. Use according to claim 21, characterised in that the antibody or antibody equivalent has a toxic marker.

23. Use according to claim 22, characterised in that the marker is a radioisotope.

24. Use according to one of claims 19 and 20, characterised in that the agent is a tissue inhibiter or the like, preferably synthetic, of metalloproteinase specific to stromelysine-3.

25. Use according to one of claims 19 and 20, characterised in that the agent modifies the activity of stromelysine-3.

26. Use according to one of claims 19 and 20, characterised in that the agent prevents the expression of the stromelysine-3 gene.

27. Use according to one of claims 19 and 20, characterised in that the agent prevents the maturation of the pre-proprotein into stromelysine-3.

28. Method for producing a vaccine comprising mixing a peptide as defined in claim 12, or a virus containing a nucleotide sequence coding for such a peptide, conjointly with an appropriate support medium therefor.
